# EUROPEAN PATENT SPECIFICATION

(11) **EP 2 722 905 B1**
(45) Date of publication and mention of the grant of the patent: **19.09.2018**
(21) Application number: 12800150.0
(22) Date of filing: 11.06.2012
(51) Int. Cl.: C09K 11/06, H01L 51/50, C07C 15/38, C07D 209/86, C07D 213/16, C07D 307/80, C07D 401/10, C07D 487/04

(54) **LIGHT EMITTING ELEMENT MATERIAL AND LIGHT EMITTING ELEMENT**
MATERIAL FÜR EIN LICHTEMITTIERENDES ELEMENT UND LICHTEMITTIERENDES ELEMENT
MATIÈRE D'ÉLÉMENT ÉLECTROLUMINESCENT ET ÉLÉMENT ÉLECTROLUMINESCENT

(30) Priority: 15.06.2011 JP 2011132851
(43) Date of publication of application: 23.04.2014
(73) Proprietor: Toray Industries, Inc., Tokyo 103-8666 (JP)
(72) Inventor: UEOKA, Koji, Otsu-shi, Shiga 520-8558 (JP); NAGAO, Kazumasa, Otsu-shi, Shiga 520-8558 (JP); SUGIMOTO, Kazunori, Otsu-shi, Shiga 520-8558 (JP); TOMINAGA, Tsuyoshi, Otsu-shi, Shiga 520-8558 (JP)
(74) Representative: Hoefer & Partner Patentanwälte mbB
(86) International application number: PCT/JP2012/064872
(87) International publication number: WO 2012/173073

(56) References cited:
- JP-A- 2006 298 793
- JP-A- 2009 004 351
- JP-A- 2010 061 824
- JP-A- 2010 061 824
- JP-A- 2011 173 972
- JP-A- 2012 079 892
- US-A1- 2006 251 925
- US-A1- 2011 309 343
- WANG ET AL LEVASON BILL ET AL: "Luminescence and electroluminescence of Al(III), B(III), Be(II) and Zn(II) complexes with nitrogen donors", COORDINATION CHEMISTRY REVIEWS, ELSEVIER SCIENCE, AMSTERDAM, NL, vol. 215, no. 1, 1 May 2001 (2001-05-01), pages 79-98, XP027216778, ISSN: 0010-8545 [retrieved on 2001-05-01]

## Description

### [Technical Field]

The present invention relates to a light emitting device which can convert electric energy into light. More particularly, the present invention relates to a light emitting device which can be utilized in the fields of display devices, flat panel displays, backlights, illuminations, interiors, signs, billboards, electrophotographic machines, and light signal generators.

### [Background Art]

In recent years, there have been intensive researches performed on an organic thin-film light emitting device in which electrons injected from a cathode and holes injected from an anode emit light when they are recombined in an organic fluorescent body held by both electrodes. This light emitting device has been attracting attention because of such a feature that it is thin and capable of emitting high-luminance light under a low driving voltage and emitting multicolor light through selection of a fluorescent material. Such researches have been studied by many research institutes since C. W. Tang et al. of Kodak Co., Ltd. showed that an organic thin-film device emits light at high luminance.

Since the organic thin-film light emitting device can afford a variety of light-emitted colors by using various fluorescent materials in an emissive layer, and studies of practical realization for displays and the like have been intensively performed. Among emissive materials emitting three primary colors, a research on a green emissive material is most advanced and, currently in a red emissive material and a blue emissive material, a research has been intensively performed aiming at improvement in properties.

It is necessary that the organic thin-film light emitting device satisfies an improvement in luminance efficiency, reduction in a driving voltage and an improvement in durability. It becomes impossible to output an image requiring high luminance due to low luminance efficiency, leading to an increase in the amount of power consumed for outputting desired luminance. For example, in order to improve the luminance efficiency, there have been developed emissive materials and electron transporting materials which contain pyrene as a basic skeleton (see, for example, Patent Literatures 1 to 4). There have also been disclosed techniques of doping a material used as an electron transporting layer with an alkali metal (see, for example, Patent Literatures 5 to 6).

Patent Literature 7 teaches a color converting composition with high efficiency of color conversion and high color purity. The color converting composition converts light emission from a light emitter to light of longer wavelength, contains a compound having a pyrromethene frame expressed in a specific general formula or its metal complex, and a light-absorbing compound having a long wavelength end of light absorption in a region of 400 nm to 600 nm.

Patent Literature 8 discloses an organic electroluminescence device having high emission luminance, high heat resistance, excellent high-temperature storage stability, and a long lifetime, and an aromatic amine derivative for realizing the device. Provided are a novel aromatic amine derivative having a specific structure and an organic electroluminescence device including: a cathode; an anode; and one or multiple organic thin film layers including at least a light-emitting layer, the one or multiple organic thin film layers being interposed between the cathode and the anode, in which at least one layer of the one or multiple organic thin film layers contains the aromatic amine compound alone or as a component of a mixture.

### [Citation List]

### [Patent Literature]

[Patent Literature 1]
   Japanese Unexamined Patent Publication (Kokai) No. 2007-131723
[Patent Literature 2]
   Japanese Unexamined Patent Publication (Kokai) No. 2010-056190
[Patent Literature 3]
   International Publication WO 2007/29798 pamphlet
[Patent Literature 4]
   International Publication WO 2008/108256 pamphlet
[Patent Literature 5]
   International Publication WO 2010/113743 pamphlet
[Patent Literature 6]
   International Publication WO 2010/001817 pamphlet
[Patent Literature 7]
   JP 2010-061824 A
[Patent Literature 8]
   US 2006/251925 A1

### [Summary of Invention]

### [Technical Problem]

However, there are a few blue emissive materials capable of providing a device which enables high-efficiency light emission and low-voltage driving, and is also excellent in durability and exhibits high reliability, particularly regarding a blue light emitting device.

Even when a compound used in an electron transporting layer is improved, conventionally known combinations are insufficient in realization of both of low-voltage driving and durability.

An object of the present invention is to solve such problems of the prior art, and to provide a light emitting device material capable of providing an organic thin-film light emitting device which enables high-efficiency light emission and low-voltage driving, and is also excellent in durability; and a light emitting device using the same.

### [Solution to Problem]

The present invention is defined in the appended claims and is directed to a light emitting device material containing a compound represented by the following general formula (1): 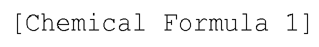 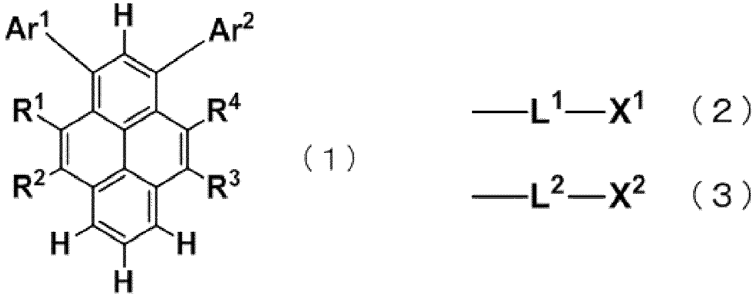 wherein R¹ to R⁴ may be the same or different, and are each selected from the group consisting of hydrogen, an alkyl group, a cycloalkyl group, a heterocyclic group, an alkenyl group, a cycloalkenyl group, an alkynyl group, an alkoxy group, an alkylthio group, an arylether group, an arylthioether group, an aryl group, a heteroaryl group, halogen, a carbonyl group, a carboxyl group, an oxycarbonyl group, a carbamoyl group, an amino group, a silyl group, and -P(=O)R⁵R⁶; R⁵ and R⁶ are each an aryl group or a heteroaryl group, and adjacent substituents may form a ring; Ar¹ is a group represented by the general formula (2); Ar² is a group represented by the general formula (3); L¹ and L² are each a single bond, an arylene group, or a heteroarylene group, and each may be the same or different, provided that when L¹ and L² are each a naphthalenylene group, L¹ and L² each is not linked to pyrene at the 1-position of a naphthalene ring; and X¹ and X² are each an aryl group or a heteroaryl group, and each may be the same or different, provided that when L¹ is a single bond, X¹ is not a 1-naphthyl group and, when L² is a single bond, X² is not a 1-naphthyl group, wherein at least one of X¹ and X² is an aromatic heterocyclic group containing electron-accepting nitrogen.

The present invention is also directed to a light emitting device comprising an anode, a cathode, and an organic layer existing between the anode and the cathode, the light emitting device emitting light from electric energy, wherein the organic layer contains the above-mentioned light emitting device material.

### [Advantageous Effects of Invention]

According to the present invention, it is possible to provide an organic electroluminescence device which enables high-efficiency light emission and low-voltage driving, and is also excellent in durability.

### [Description of Embodiments]

The compound represented by the general formula (1) will be described in detail below.

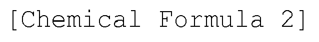

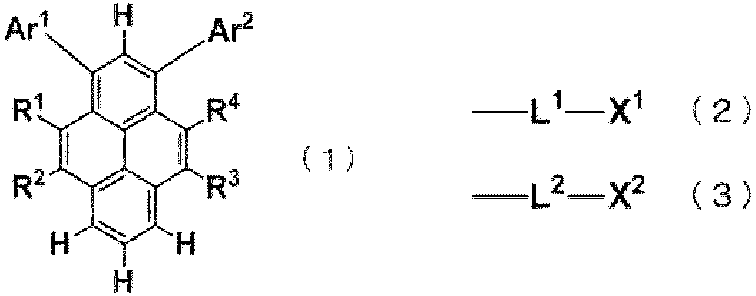

R¹ to R⁴ may be the same or different, and are each selected from the group consisting of hydrogen, an alkyl group, a cycloalkyl group, a heterocyclic group, an alkenyl group, a cycloalkenyl group, an alkynyl group, an alkoxy group, an alkylthio group, an arylether group, an arylthioether group, an aryl group, a heteroaryl group, halogen, a carbonyl group, a carboxyl group, an oxycarbonyl group, a carbamoyl group, an amino group, a silyl group, and -P(=O)R⁵R⁶. R⁵ and R⁶ are each an aryl group or a heteroaryl group, and adjacent substituents may form a ring. Ar¹ is a group represented by the general formula (2), and Ar² is a group represented by the general formula (3).

L¹ and L² are each a single bond, an arylene group, or a heteroarylene group, and each may be the same or different. Provided that when L¹ and L² are each a naphthalenylene group, L¹ and L² each is not linked to pyrene at the 1-position of a naphthalene ring. At least one of X¹ and X² is an aromatic heterocyclic group containing electron-accepting nitrogen. Provided that when L¹ is a single bond, X¹ is not a 1-naphthyl group and, when L² is a single bond, X² is not a 1-naphthyl group.

Among these substituents, hydrogen may be deuterated hydrogen.

The alkyl group represents, for example, a saturated aliphatic hydrocarbon group such as a methyl group, an ethyl group, an n-propyl group, an isopropyl group, an n-butyl group, a sec-butyl group, or a tert-butyl group, and this may or may not have a substituent. When the alkyl group is substituted, the additional substituent is not particularly limited and examples thereof include an alkyl group, an aryl group, a heteroaryl group, and the like. This respect is common to the following description. The number of carbon atoms of the alkyl group is not particularly limited, but is preferably in a range of 1 or more and 20 or less, and more preferably in a range of 1 or more and 8 or less, in view of ease of availability and cost.

The cycloalkyl group represents, for example, a saturated alicyclic hydrocarbon group such as a cyclopropyl group, a cyclohexyl group, a norbornyl group, or an adamantyl group, and it may or may not have a substituent. The number of carbon atoms of an alkyl group moiety is not particularly limited, but is preferably in a range of 3 or more and 20 or less.

The heterocyclic group represents an aliphatic ring having an atom other than carbon in the ring, such as a pyran ring, a piperidine ring, or a cyclic amide, and it may or may not have a substituent. The number of carbon atoms of the heterocyclic group is not particularly limited, but is preferably in a range of 2 or more and 20 or less.

The alkenyl group represents an unsaturated aliphatic hydrocarbon group containing a double bond, such as a vinyl group, an allyl group, or a butadienyl group, and it may or may not have a substituent. The number of carbon atoms of the alkenyl group is not particularly limited, but is preferably in a range of 2 or more and 20 or less.

The cycloalkenyl group represents an unsaturated alicyclic hydrocarbon group containing a double bond, such as a cyclopentenyl group, a cyclopentadienyl group, or a cyclohexenyl group, and it may or may not have a substituent.

The alkynyl group represents an unsaturated aliphatic hydrocarbon group containing a triple bond, such as an ethynyl group, and it may or may not have a substituent. The number of carbon atoms of the alkynyl group is not particularly limited, but is preferably in a range of 2 or more and 20 or less.

The alkoxy group represents a functional group to which an aliphatic hydrocarbon group is linked via an ether bond, such as a methoxy group, an ethoxy group, or a propoxy group, and the aliphatic hydrocarbon group may or may not have a substituent. The number of carbon atoms of the alkoxy group is not particularly limited, but is preferably in a range of 1 or more and 20 or less

The alkylthio group is a group resulting from replacement of an oxygen atom of the ether bond of an alkoxy group by a sulfur atom. The hydrocarbon group in the alkylthio group may or may not have a substituent. The number of carbon atoms of the alkylthio group is not particularly limited, but is preferably in a range of 1 or more and 20 or less.

The arylether group represents a functional group to which an aromatic hydrocarbon group is linked via an ether bond, such as a phenoxy group, and the aromatic hydrocarbon group may or may not have a substituent. The number of carbon atoms of the arylether group is not particularly limited, but is preferably in a range of 6 or more and 40 or less.

The arylthioether group is a group resulting from the replacement of an oxygen atom of the ether bond of an arylether group by a sulfur atom. The aromatic hydrocarbon group in the arylether group may or may not have a substituent. The number of carbon atoms of the arylether group is not particularly limited, but is preferably in a range of 6 or more and 40 or less.

The aryl group represents an aromatic hydrocarbon group such as a phenyl group, a naphthyl group, a phenanthryl group, a terphenyl group, or a fluoranethenyl. The aryl group may or may not have a substituent. The number of carbon atoms of the aryl group is not particularly limited, but is preferably in a range of 6 or more and 40 or less.

The heteroaryl group represents a cyclic aromatic group having one atom or a plurality of atoms other than carbon in the ring, such as a furanyl group, a thiophenyl group, a pyridyl group, a quinolinyl group, an isoquinolinyl group, a pyrazinyl group, a pyrimidyl group, a naphthyridyl group, a benzofuranyl group, a benzothiophenyl group, an indolyl group, a dibenzofuranyl group, a dibenzothiophenyl group, or a carbazolyl group, and it may or may not be substituted. The number of carbon atoms of the heteroaryl group is not particularly limited, but is preferably in a range of 2 or more and 30 or less.

The halogen represents an atom selected from fluorine, chlorine, bromine, and iodine.

The carbonyl group, the carboxyl group, the oxycarbonyl group, the carbamoyl group, and the amino group may or may not have a substituent. Examples of the substituent include an alkyl group, a cycloalkyl group, an aryl group, and a heteroaryl group, and these substituents may be further substituted.

The silyl group represents a functional group in which an organic group such as an alkyl group, a cycloalkyl group, an alkoxy group, or an aryl group is linked to a silicon atom, such as a trimethylsilyl group, and this may or may not have a substituent. The number of carbon atoms of the silyl group is not particularly limited, but is preferably in a range of 3 or more and 20 or less. The number of silicon is usually in a range of 1 or more and 6 or less.

Regarding the group represented by -P(=O)R⁵R⁶, R⁵ and R⁶ are each an aryl group or a heteroaryl group, and adjacent substituents may form a ring. When adjacent substituents R⁵ and R⁶ form a ring, R⁵ and R⁶ may be combined with each other to form a conjugated or non-conjugated fused ring. The constituent element of the fused ring may include, in addition to carbon, atoms selected from nitrogen, oxygen, sulfur, phosphorus, and silicon. The fused ring may further be fused with another ring.

The arylene group represents a divalent group derived from an aromatic hydrocarbon group such as a phenyl group, a naphthyl group, a biphenyl group, a phenanthryl group, or a terphenyl group, and this may or may not have a substituent. The number of carbon atoms of the arylene group is not particularly limited, but is preferably in a range of 6 or more and 40 or less.

The heteroarylene group represents a divalent group derived from a cyclic aromatic group having one atom or a plurality of atoms other than carbon in the ring, such as a pyridyl group, a quinolinyl group, a pyrazinyl group, a naphthyridyl group, a dibenzofuranyl group, a dibenzothiophenyl group, or a carbazolyl group, and this may or may not have a substituent. The number of carbon atoms of the heteroarylene group is not particularly limited, but is preferably in a range of 2 or more and 30 or less, including a substituent.

In the compound represented by the general formula (1), when a hydrogen atom is located at all 6-, 7-, and 8-positions of the pyrene skeleton, it is possible to increase the interaction between pyrene skeletons. This effect facilitates intermolecular delivery of electrons to exhibit high electron transportability, and thus enabling a decrease in driving voltage of the obtained light emitting device. When hydrogen atoms at both 1- and 3-positions of the pyrene skeleton are substituted with an aryl group or a heteroaryl group, thermal stability is improved to obtain a stable film, and thus enabling an improvement in durability of the obtained light emitting device.

When Ar¹ and Ar² are each a different group, as molecular symmetry decreases, the glass transition temperature increases, and thus improving amorphous property of the obtained thin film. If a thin film has high amorphous property, a change in film structure and crystallization are less likely to arise during current driving of the obtained light emitting device, and thus suppressing an increase in driving voltage and a decrease in luminance of the light emitting device. Therefore, it is preferred that Ar¹ and Ar² are each different groups.

L¹ and L² are each may or may not have a substituent.

L¹ and L² are each preferably an arylene group, since thermal stability enhances. L¹ and L² are each more preferably a single bond, a phenylene group, a biphenylene group, or a naphthalenylene group. Provided that the compound having a structure in which a pyrene skeleton is directly linked to the 1-postion of the naphthalene ring is likely to cause a condensation reaction as shown in the following formula, through heat.

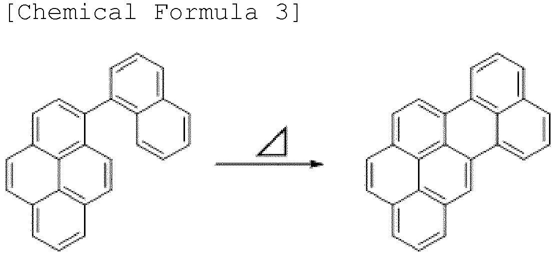

In the production of the organic thin-film light emitting device, an organic compound used as a material is subjected to the sublimation refining or deposition step. If the above-mentioned condensation reaction occurs in this step, it becomes impossible to obtain characteristics inherent in the material. In order to prevent the above-mentioned condensation reaction, there is exemplified a method in which a substituent R is introduced into the naphthalene ring at the 8-position, as shown in the following formula. However, such compound causes great steric hindrance due to a substituent and may cause a decrease in interaction between pyrene skeletons, leading to deterioration of electron transportability.

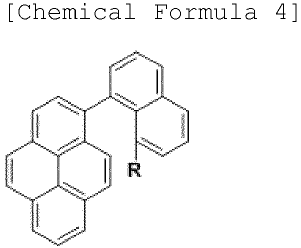

Therefore, when L¹ and L² are each a naphthalenylene group, L¹ and L² each should not be linked to the pyrene skeleton at the 1-position of the naphthalene ring. For the same reason, when L¹ is a single bond, X¹ should not be a 1-naphthyl group and, when L² is a single bond, X² should not be a 1-naphthyl group. Since the substituent in which an aromatic ring is fused with a 1-naphthyl group causes the same problem, it should not be directly linked to the pyrene skeleton, similarly. Examples of specific substituent causing such problem include a 1-phenanthryl group, a 9-phenanthryl group, a 1-anthryl group, a 9-anthryl group, a 1-pyrenyl group, and the like.

Specific examples of preferable L¹ and L² include a single bond, a 1,4-phenylene group, a 1,3-phenylene group, a 4,4'-biphenylene group, a 2,6-naphthalenylene group, a 2,8-naphthalenylene group, and the like, and include more preferably a 1,4-phenylene group.

When L¹ and L² are further substituted, the substituent is not particularly limited, and preferably an alkyl group, an aryl group, a heteroaryl group, or the like. The alkyl group is preferably a methyl group, a t-butyl group, or the like. The aryl group is preferably a phenyl group, a naphthyl group, a biphenyl group, or the like. The heteroaryl group is preferably a pyridyl group and, more specifically, a 2-pyridyl group, a 3-pyridyl group, and a 4-pyridyl group are preferable.

In an embodiment not falling under the scope of the present invention X¹ and X² are each an aryl group, each independently preferably a phenyl group, a naphthyl group, a phenanthryl group, a fluorenyl group, a fluoranthenyl group, or the like. More specific examples thereof include a phenyl group, a 1-naphthyl group, a 2-naphthyl group, a 2-fluorenyl group, a fluoranthenyl group, and the like; and more preferable examples thereof include a phenyl group, a 1-naphthyl group, a 2-naphthyl group, and the like. Provided that when L¹ is a single bond, X¹ is not a 1-naphthyl group and, when L² is a single bond, X² is not a 1-naphthyl group. When X¹ and X² are more substituted, the substituent is not particularly limited and examples thereof include an alkyl group, a phenyl group, fluorine, and the like; and specific examples thereof include a methyl group, a t-butyl group, a phenyl group, fluorine, and the like.

When X¹ and X² are each a heteroaryl group (embodiment not falling under the scope of the present invention), each independently preferably an indolyl group, a benzofuranyl group, a benzothiophenyl group, a carbazolyl group, a dibenzofuranyl group, a dibenzothiophenyl group, a pyridyl group, a quinolinyl group, an isoquinolinyl group, a benzoquinolinyl group, a quinoxalinyl group, a pyrazinyl group, a pyrimidyl group, a pyridazinyl group, a phenanthrolinyl group, an imidazopyridyl group, an imidazoquinolinyl group, an imidazoquinazolinyl group, a pyridoimidazoquinolinyl group, a triazyl group, an acridyl group, a benzoimidazolyl group, a benzooxazolyl group, a benzothiazolyl group, a carbolinyl group, or the like. Specific examples thereof include a 2-indolyl group, a 2-benzofuranyl group, a 2-benzothiophenyl group, a 9-carbazolyl group, a 2-carbazolyl group, a 3-carbazolyl group, a 2-dibenzofuranyl group, a 4-dibenzofuranyl group, a 2-dibenzothiophenyl group, a 4-dibenzothiophenyl group, a 2-pyridyl group, a 3-pyridyl group, a 4-pyridyl group, a 2-quinolinyl group, a 3-quinolinyl group, a 6-quinolinyl group, a 1-isoquinolinyl group, a 4-isoquinolinyl group, a 2-benzo[h]quinolinyl group, a 3-benzo[h]quinolinyl group, a 2-quinoxalinyl group, a 1-pyrazinyl group, a 2-pyrimidyl group, a 5-pyrimidyl group, a 3-pyridazinyl group, a 2-phenanthrolinyl group, a 3-phenanthrolinyl group, a 2-imidazo[1,2-a]pyridyl group, a 3-imidazo[1,2-a]pyridyl group, a 11-benzo[4,5]imidazo[1,2-a]quinolinyl group, a 6-benzo[4,5]imidazo[1,2-c]quinazolinyl group, a 6-pyrido[2',1':2,3]imidazo[4,5-c]quinolinyl group, a 1-triazolyl group, a 9-acridyl group, a 1-benzo[d]imidazolyl group, a 2-benzo[d]imidazolyl group, a 2-benzo[d]oxazolyl group, a 2-benzo[d]thiazolyl group, a 9-α-carbolinyl group, a 9-β-carbolinyl group, a 9-γ-carbolinyl group, a 9-δ-carbolinyl group, and the like. More preferable examples thereof include a 9-carbazolyl group, a 3-carbazolyl group, a 4-dibenzofuranyl group, a 4-dibenzothiophenyl group, a 2-pyridyl group, a 3-pyridyl group, a 4-pyridyl group, a 2-quinolinyl group, a 3-quinolinyl group, a 6-quinolinyl group, a 1-isoquinolinyl group, a 4-isoquinolinyl group, a 2-benzo[h]quinolinyl group, a 2-quinoxalinyl group, a 1-pyrazinyl group, a 5-pyrimidyl group, a 2-phenanthrolinyl group, a 1-triazoyl group, a 1-benzo[d]imidazolyl group, a 2-benzo[d]imidazolyl group, and the like. More preferable thereof include a 9-carbazolyl group, a 3-carbazolyl group, a 4-dibenzofuranyl group, a 2-pyridyl group, a 3-pyridyl group, a 4-pyridyl group, and the like.

According to an embodiment not falling under the scope of the present invention, at least one of X¹ and X² is preferably an aryl group since thermal stability enhances. At least one of X¹ and X² is preferably a carbazolyl group, a dibenzofuranyl group, or a dibenzothiophenyl group since amorphous property is enhanced by a bulky aromatic heterocyclic group, resulting in enhanced thin-film stability. The carbazolyl group, the dibenzofuranyl group, and the dibenzothiophenyl group may or may have not a substituent.

According to the present invention, at least one of X¹ and X² is preferably an aromatic heterocyclic group containing electron-accepting nitrogen since high electron injectability/transportability is exhibited by enhancing electron-accepting properties.

As used herein, electron-accepting nitrogen represents a nitrogen atom which forms a multiple bond with an adjacent atom. Since the nitrogen atom has high electronegativity, the multiple bond has electron-accepting property. Therefore, an aromatic heterocycle containing electron-accepting nitrogen has high electron affinity.

The aromatic heterocyclic group containing electron-accepting nitrogen represents a cyclic aromatic group having at least one or a plurality of electron-accepting nitrogen atoms as atoms other than carbon among heteroaryl groups. Specific examples thereof include a pyridyl group, a quinolinyl group, an isoquinolinyl group, a quinoxalinyl group, a pyrazinyl group, a pyrimidyl group, a pyridazinyl group, a phenanthrolinyl group, an imidazopyridyl group, a triazyl group, an acridyl group, a benzoimidazolyl group, a benzooxazolyl group, a benzothiazolyl group, and the like. The aromatic heterocyclic group containing electron-accepting nitrogen may or may not have a substituent. The number of electron-accepting nitrogen contained in the aromatic heterocyclic group containing electron-accepting nitrogen is not particularly limited, but is preferably in a range of 1 or more and 3 or less. The number of carbon atoms of aromatic heterocyclic group containing electron-accepting nitrogen is not particularly limited, but is preferably in a range of 2 or more and 30 or less.

The linking position of the aromatic heterocyclic group containing electron-accepting nitrogen may be any position. For example, in the case of a pyridyl group, the position may be any of a 2-pyridyl group, 3-pyridyl group, and a 4-pyridyl group.

Provided that when both X¹ and X² are 2-pyridyl groups, durability of the obtained device may deteriorate. Therefore, when X¹ is a 2-pyridyl group, X² is preferably a group selected from groups other than a 2-pyridyl group.

When the aromatic heterocyclic group containing electron-accepting nitrogen is further substituted, examples of the substituent include, but are not particularly limited to, an alkyl group, an aryl group, a heteroaryl group, and the like.

The pyrene compound represented by formula (I) can be synthesized by the following methods. Examples of the method for introducing an aryl group and a heteroaryl group into the pyrene skeleton include, but are not limited to, a method using a coupling reaction between a halogenated pyrene derivative and boric acid or boric acid ester of an aryl group and a heteroaryl group in the presence of a palladium or nickel catalyst.

Examples of the method in which an aryl group or a heteroaryl group is introduced into the pyrene skeleton at the 1- and 3-positions in a position-selective manner include the following method. First, using a known method (see, for example, International Publication WO2008/108256 pamphlet), a group selected from an aryl group and a heteroaryl group in a position-selective manner is introduced into the pyrene skeleton, which is substituted with a t-butyl group at the 7-position, at the 1- and 3-positions in a position-selective manner. Then, the t-butyl group is substituted with a hydrogen atom at the 7-position by heating in an appropriate solvent, together with an acid. Examples of the acid to be used herein include, but are not limited to, strong acidic polymers (see "Journal of Organic Chemistry", (USA), 1991, Vol. 56, No. 3, pp.1334-1337) such as Nafion-H; organic acid such as trifluoromethanesulfonic acid; and Lewis acids such as aluminum trichloride.

Specific examples of the above-mentioned pyrene compound represented by the general formula (1) include, but are not particularly limited, the followings. The following compounds do not fall under the scope of the present invention: 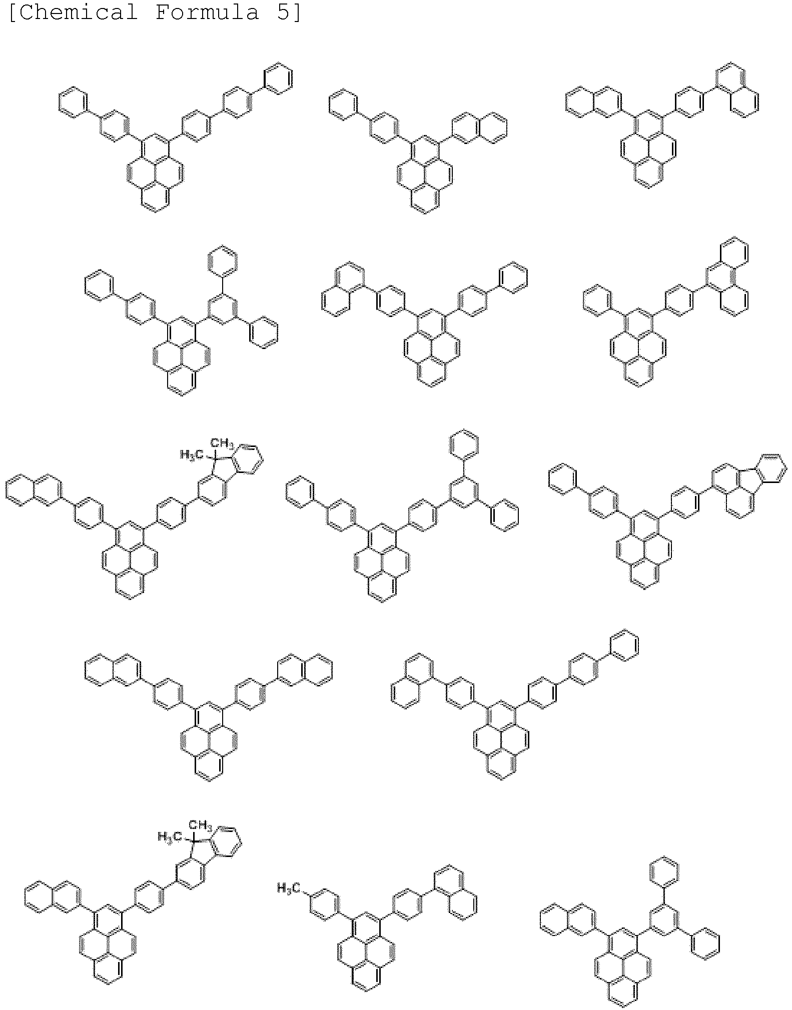 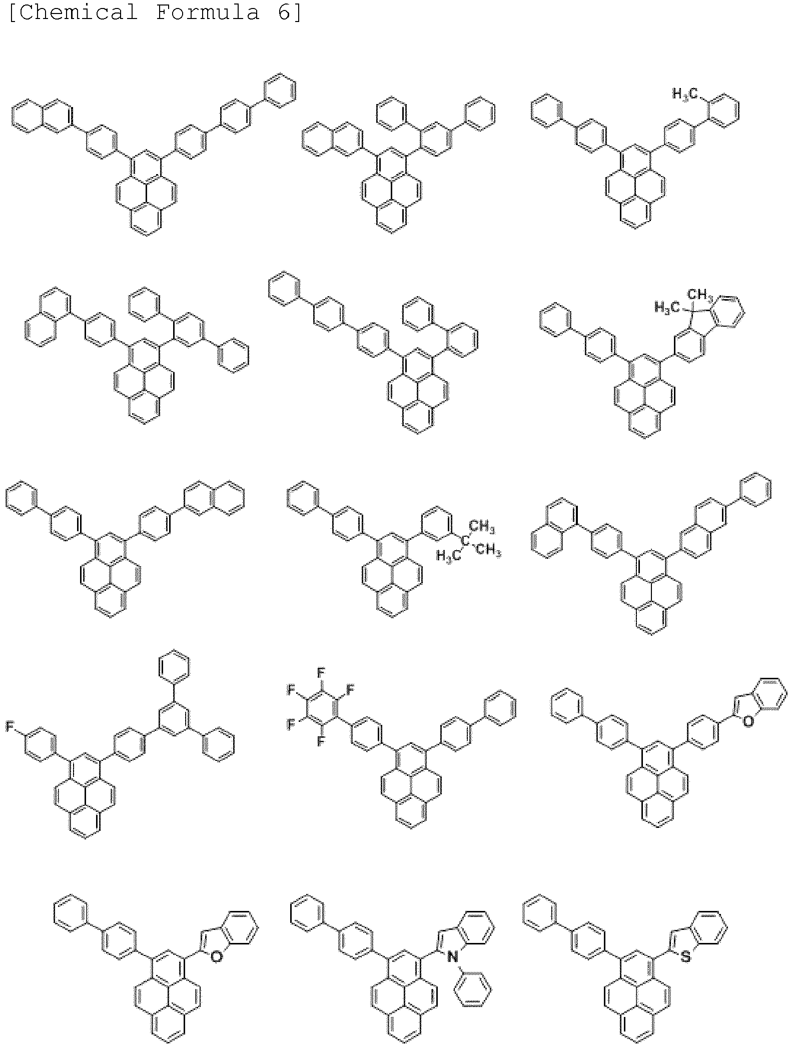 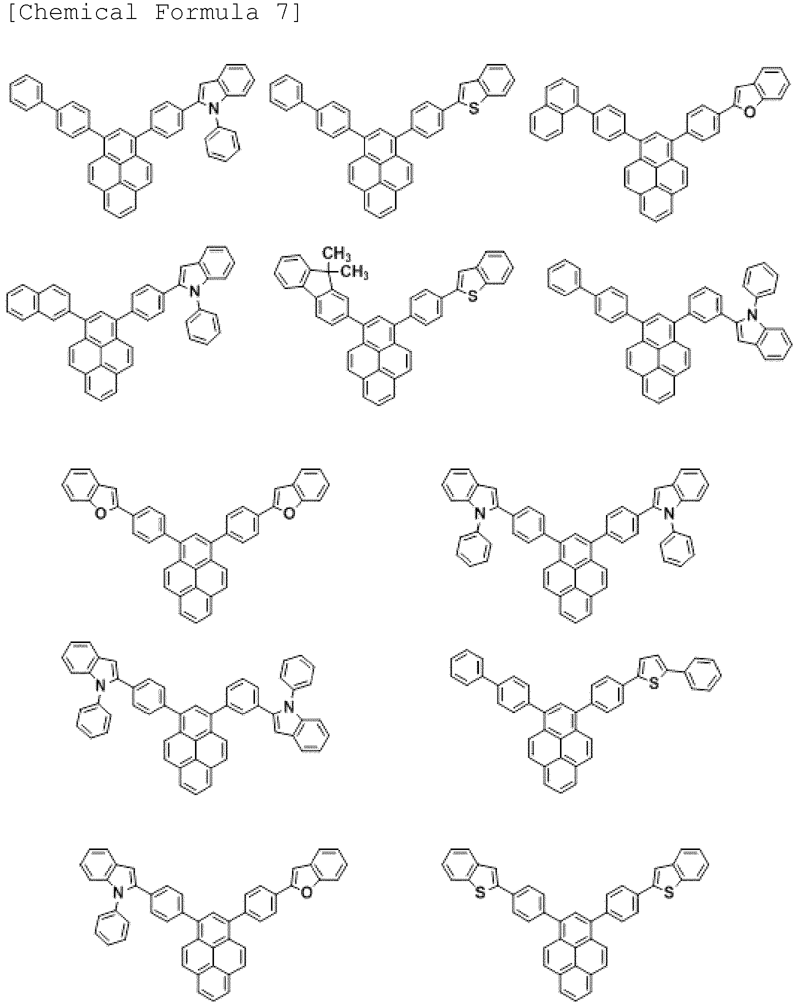 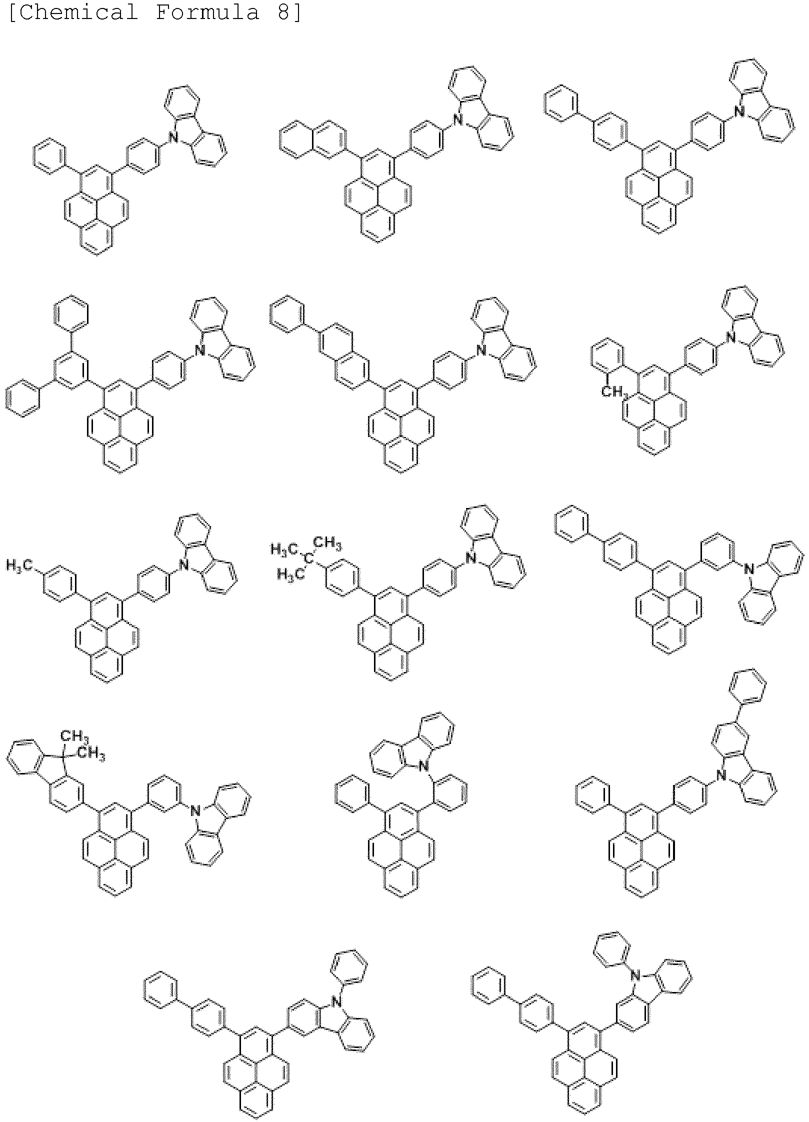 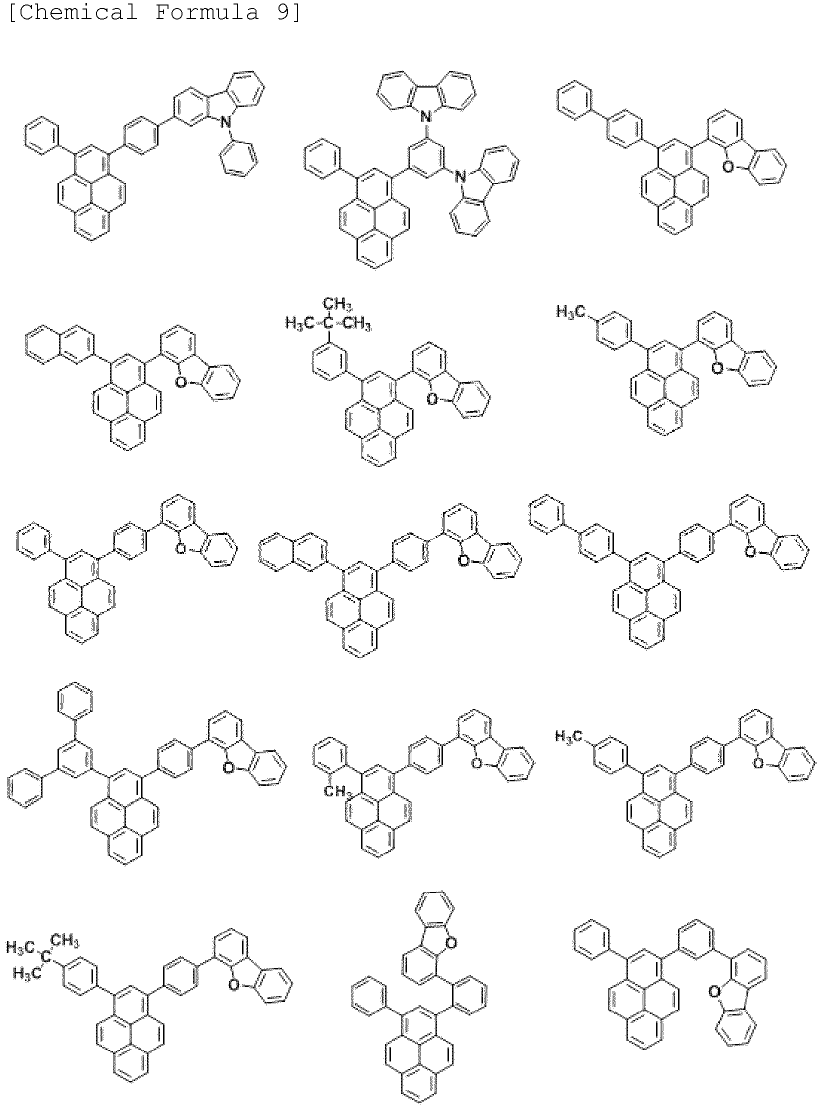 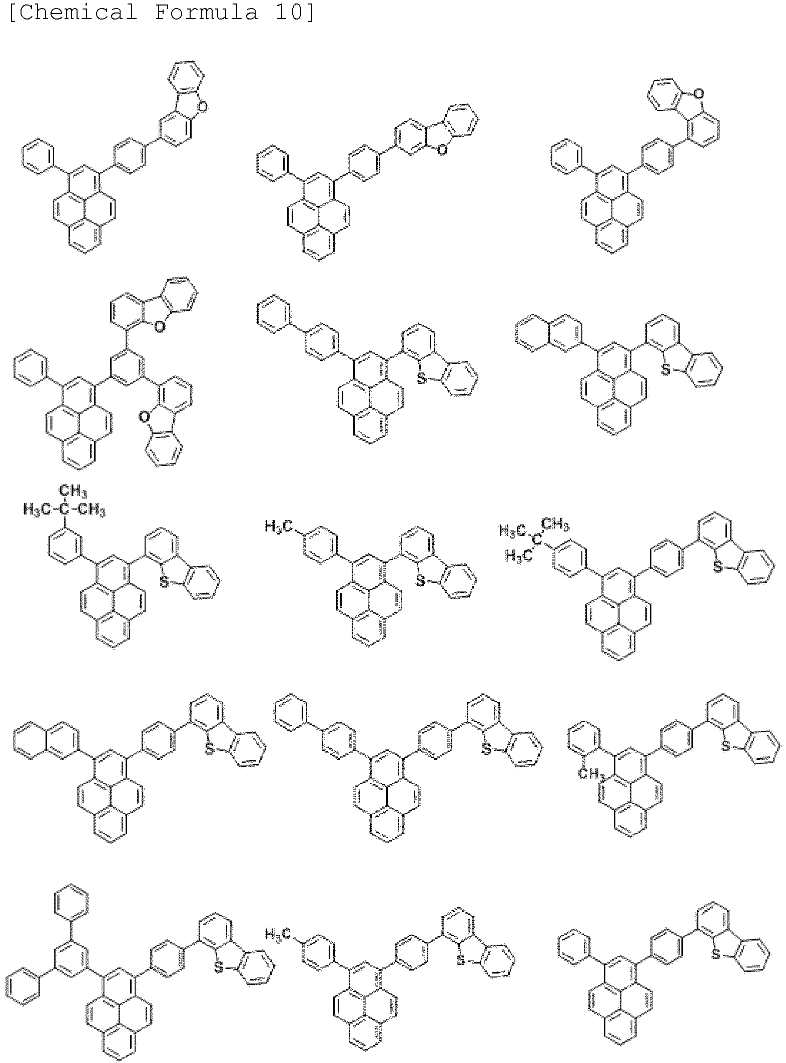 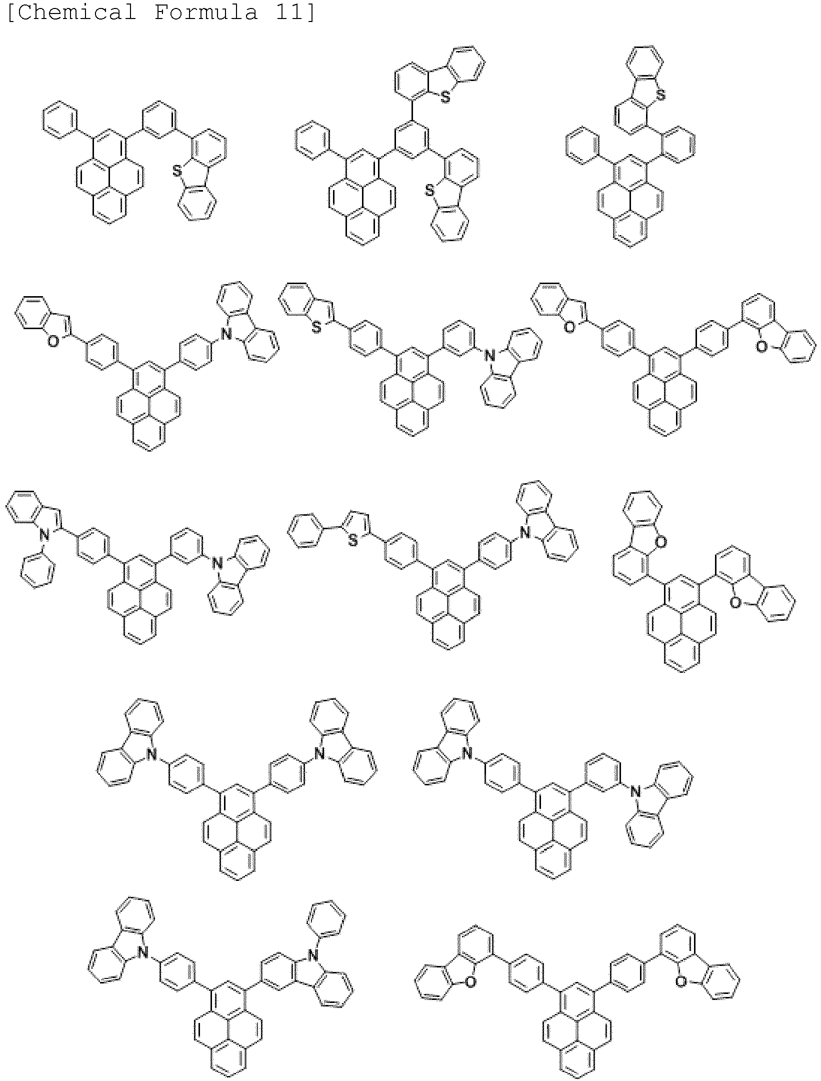 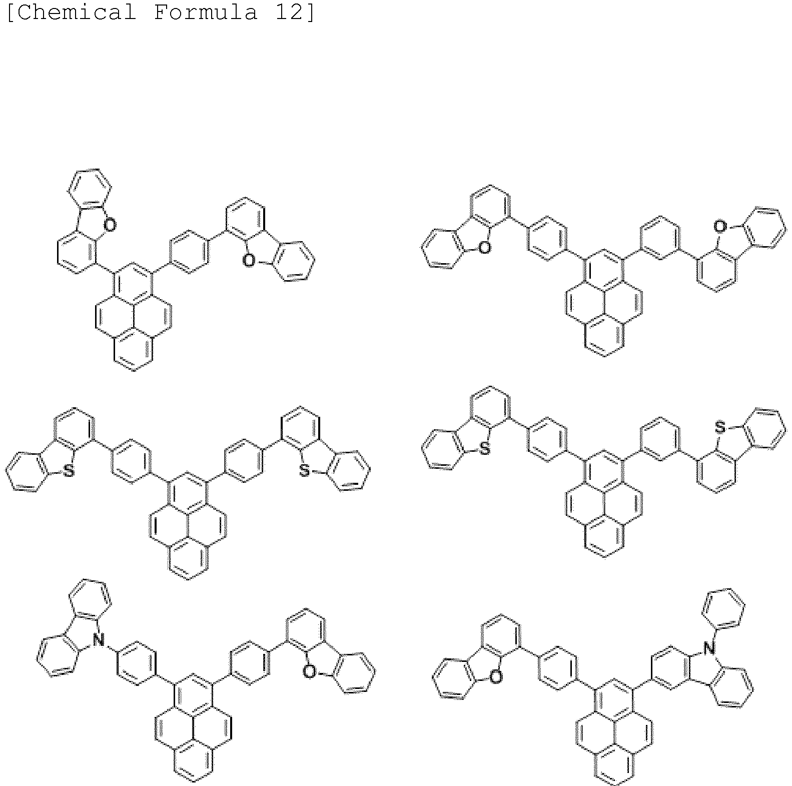 The following compounds fall under the scope of the present invention:

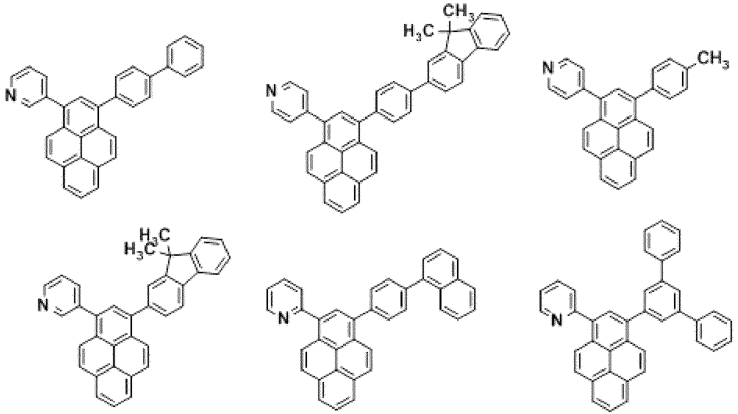

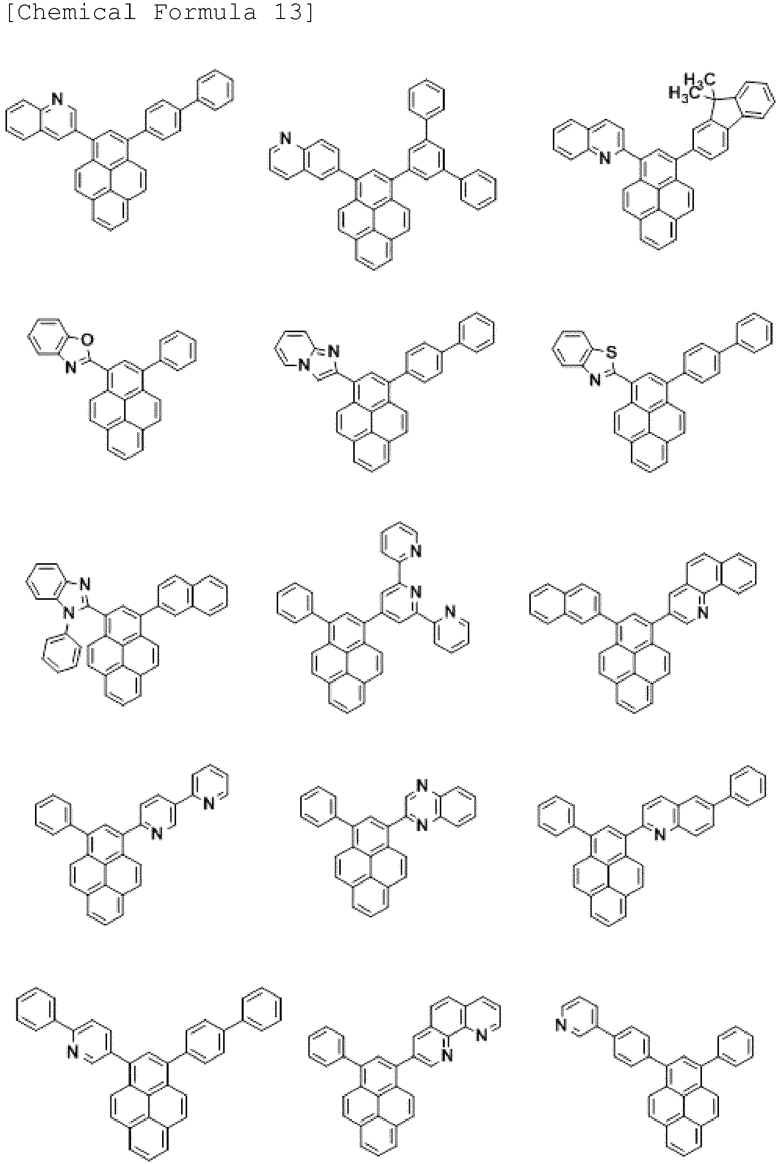

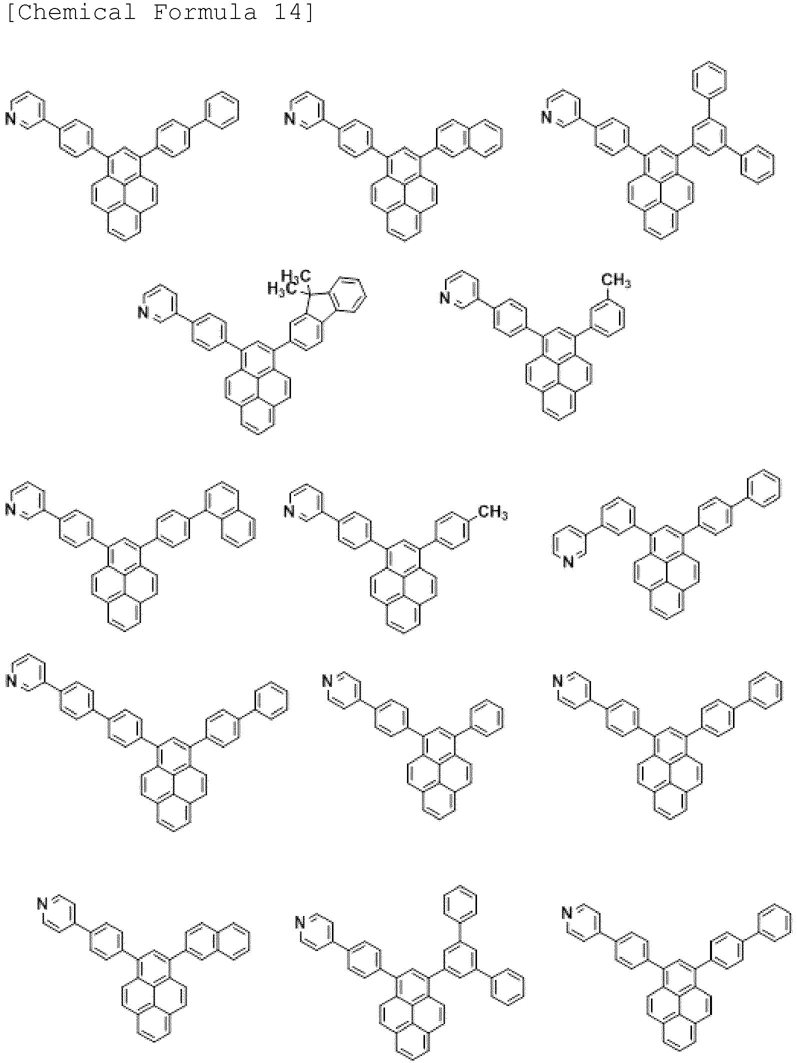

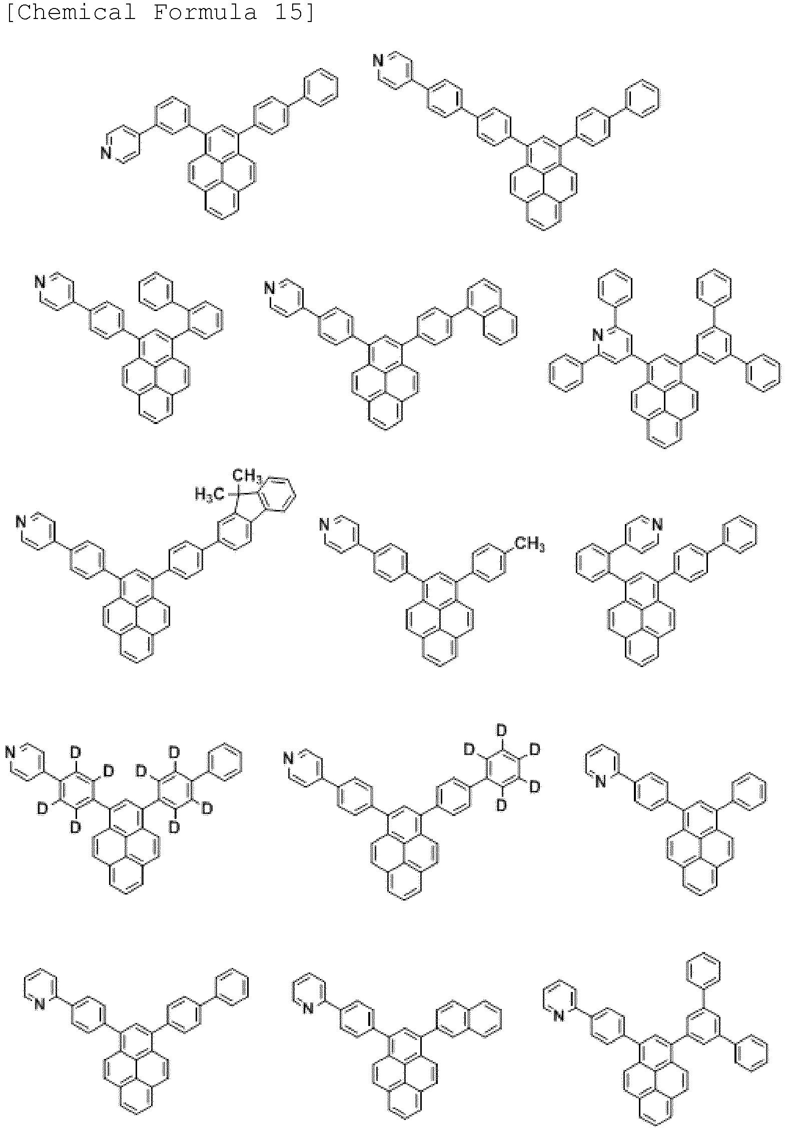

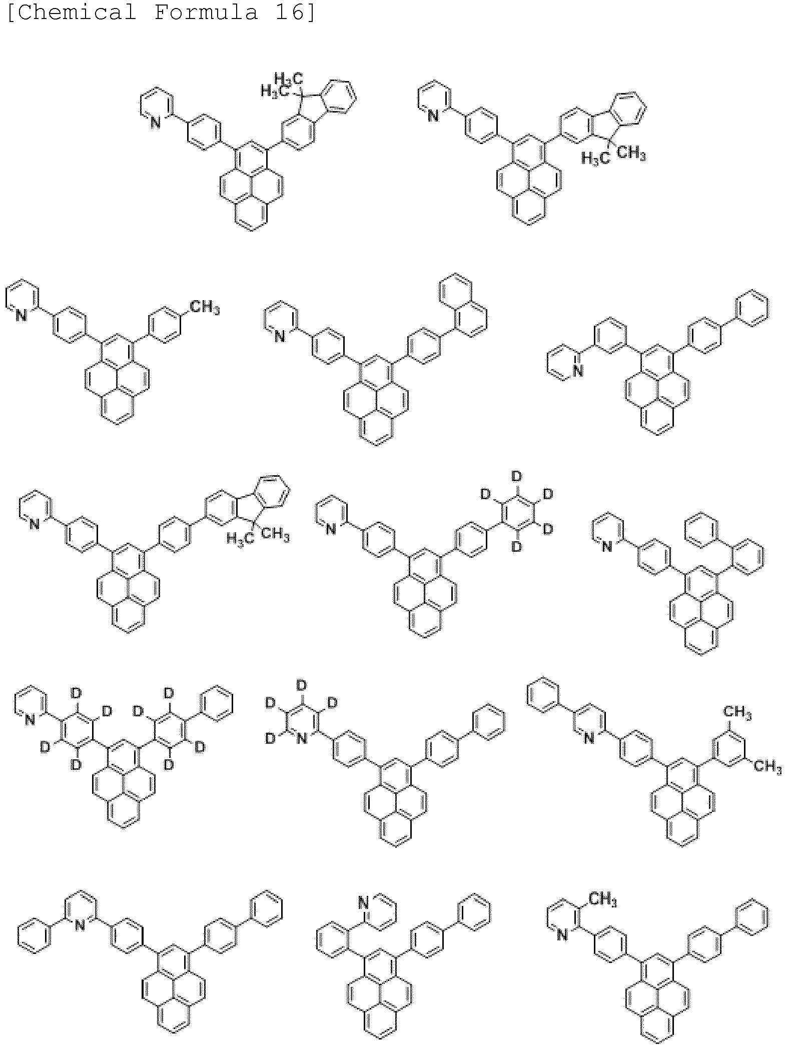

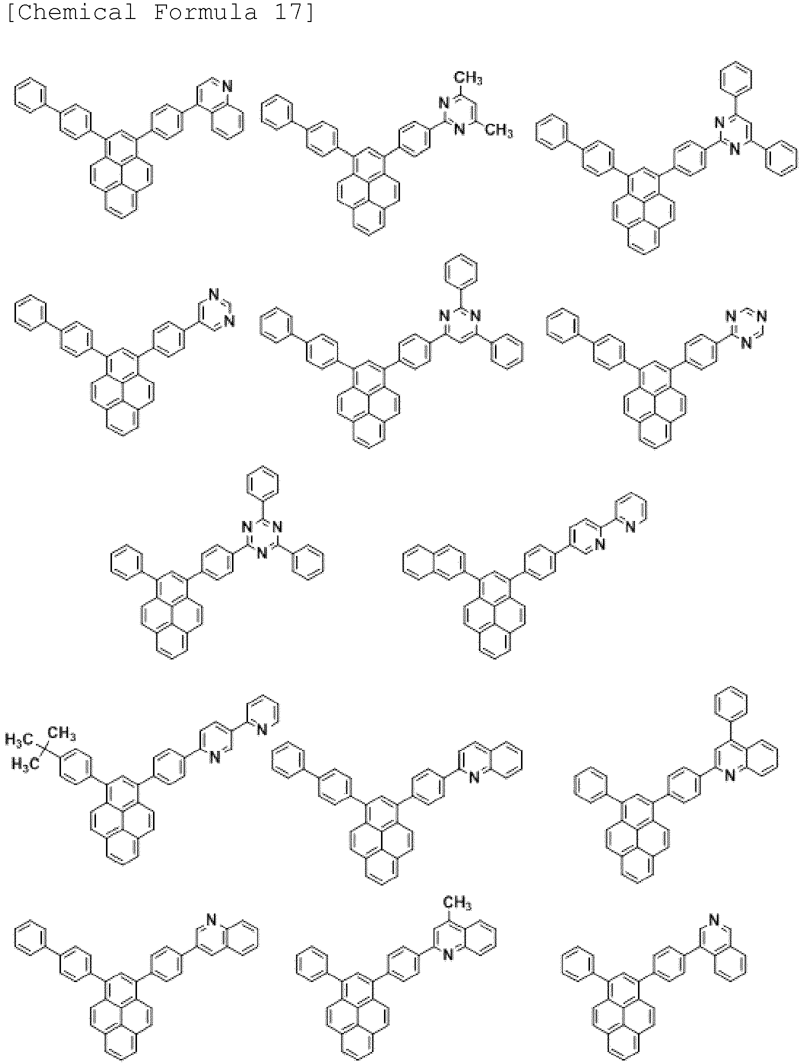

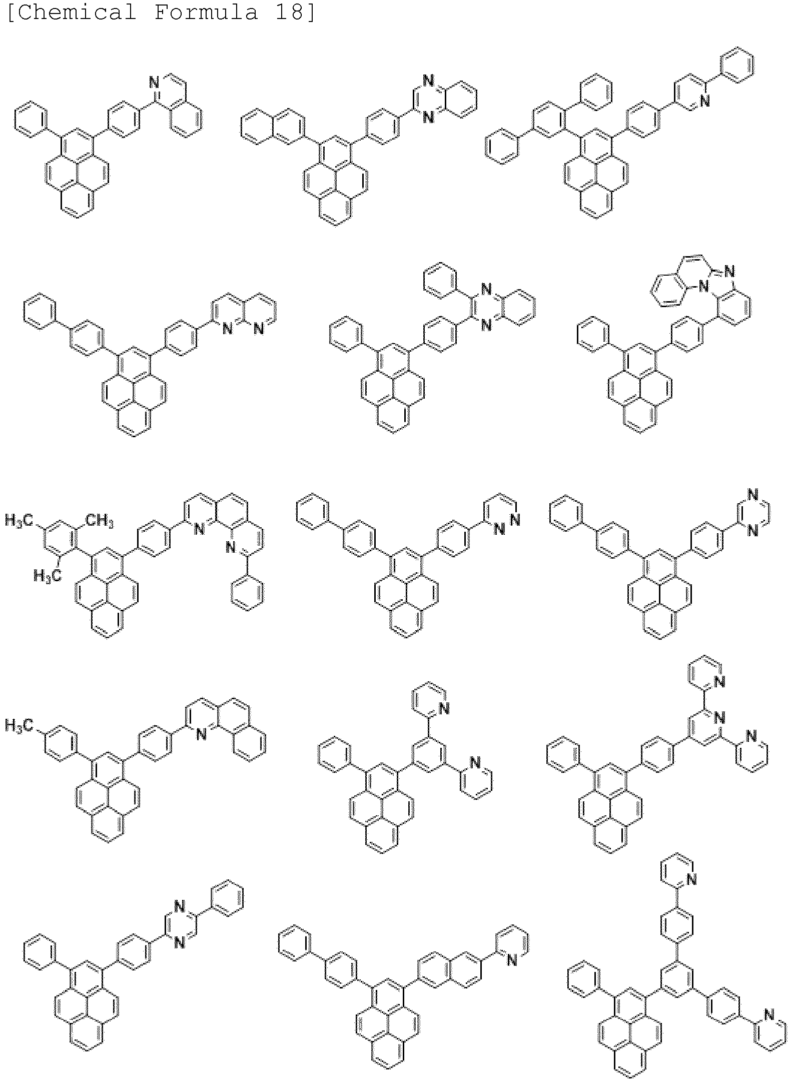

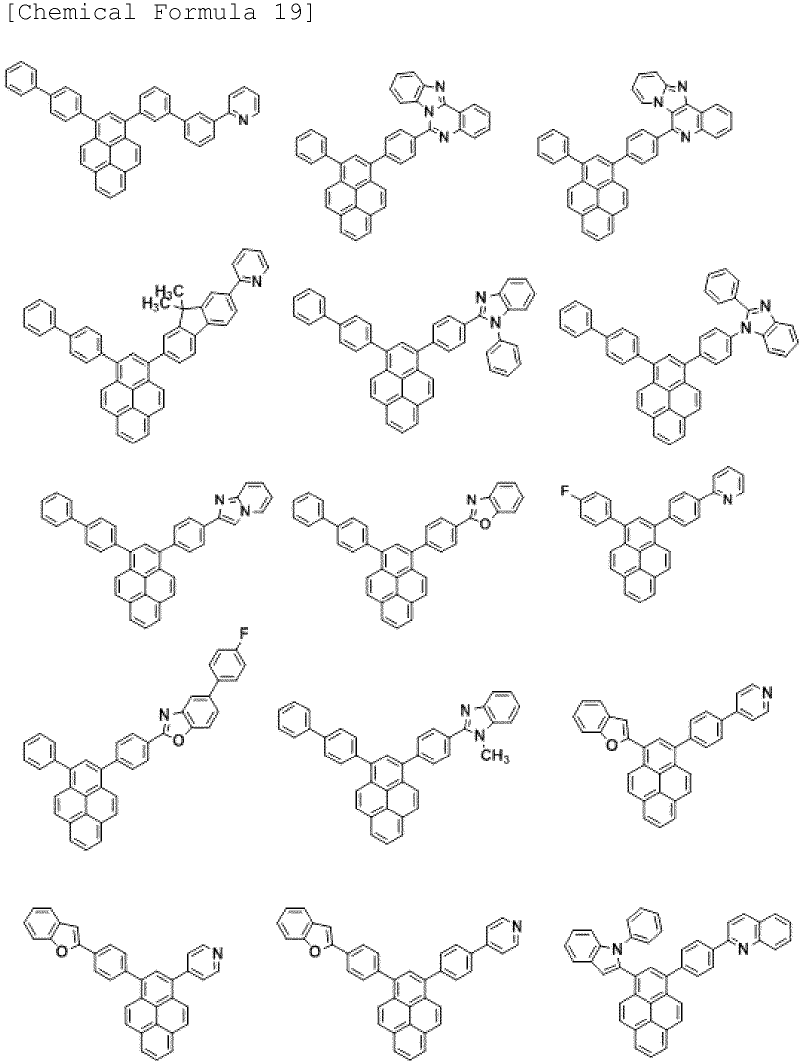

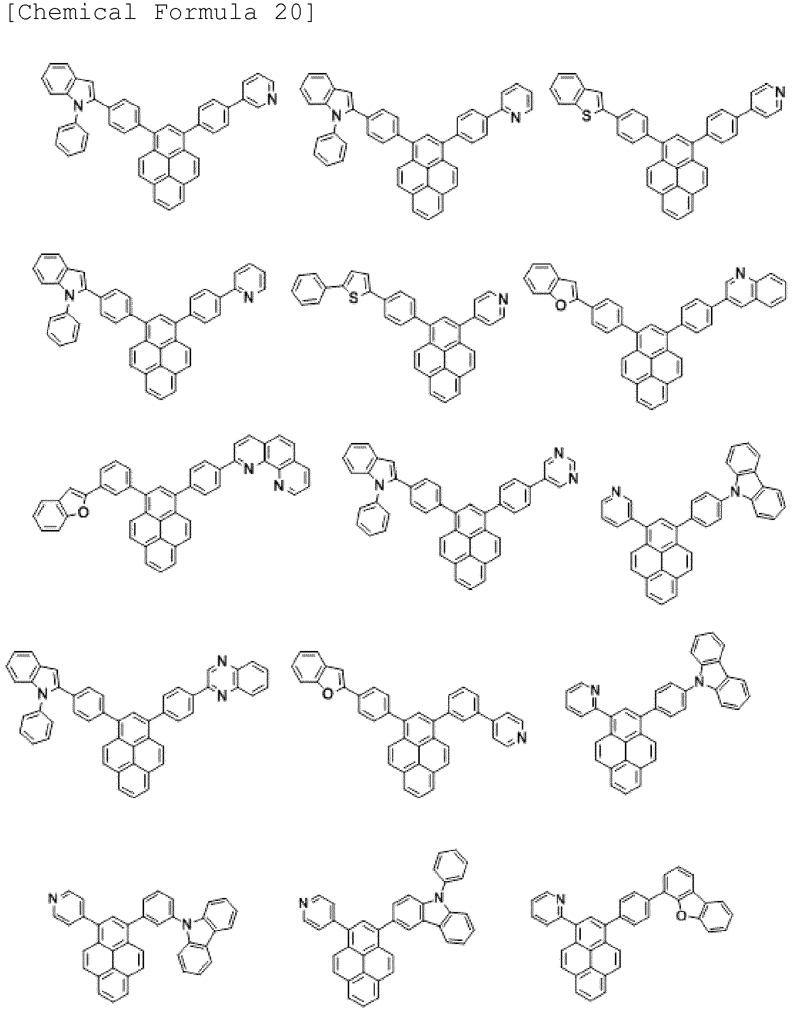

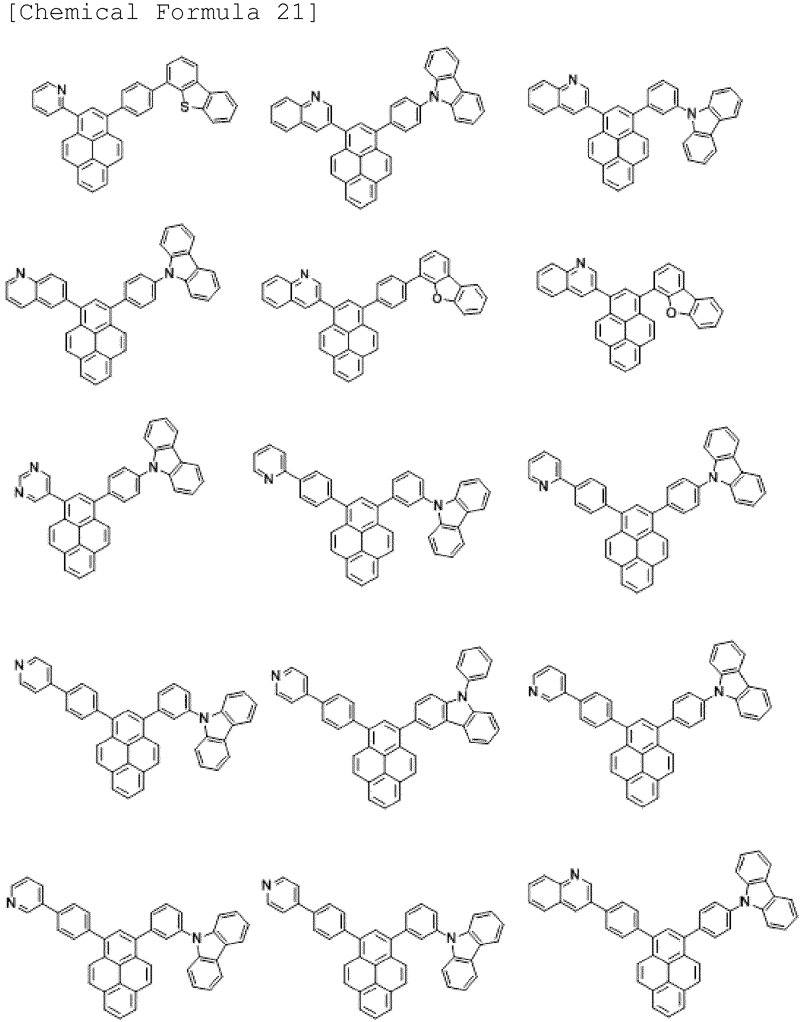

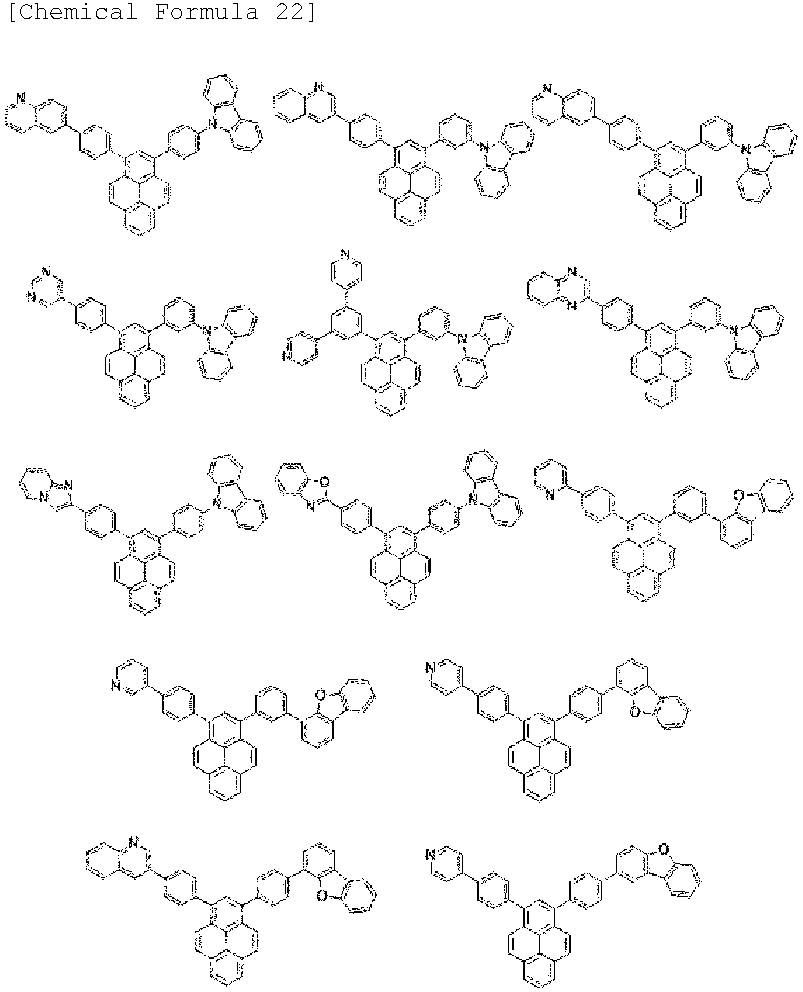

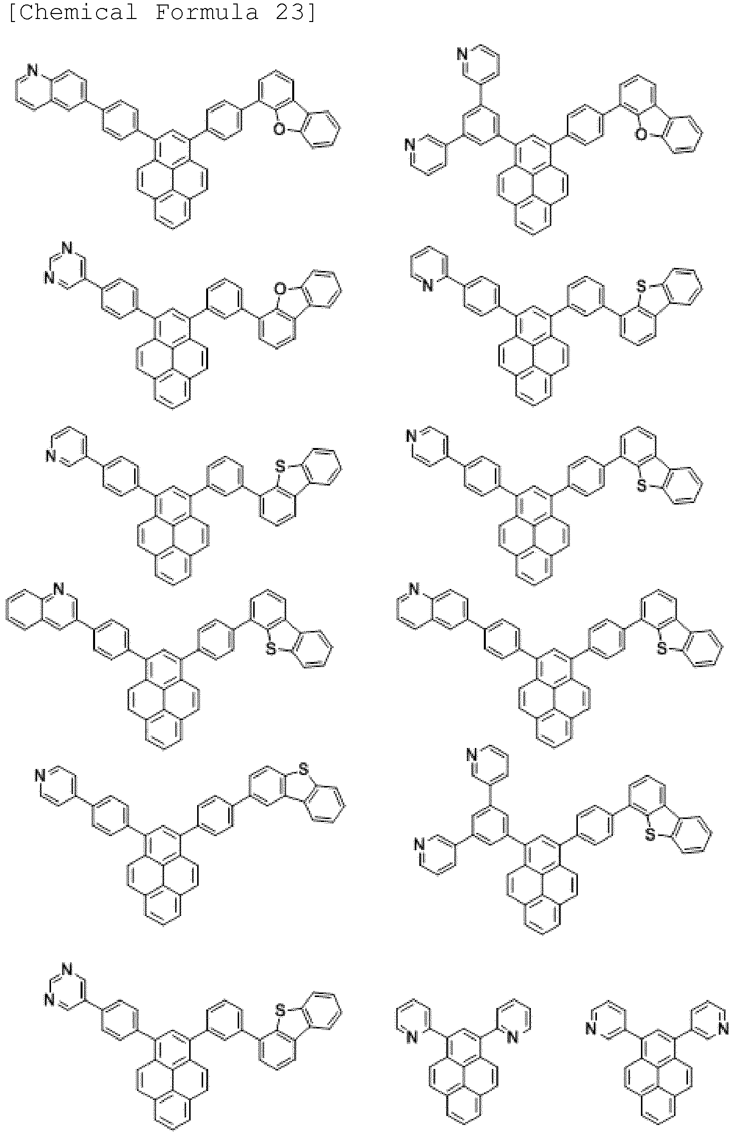

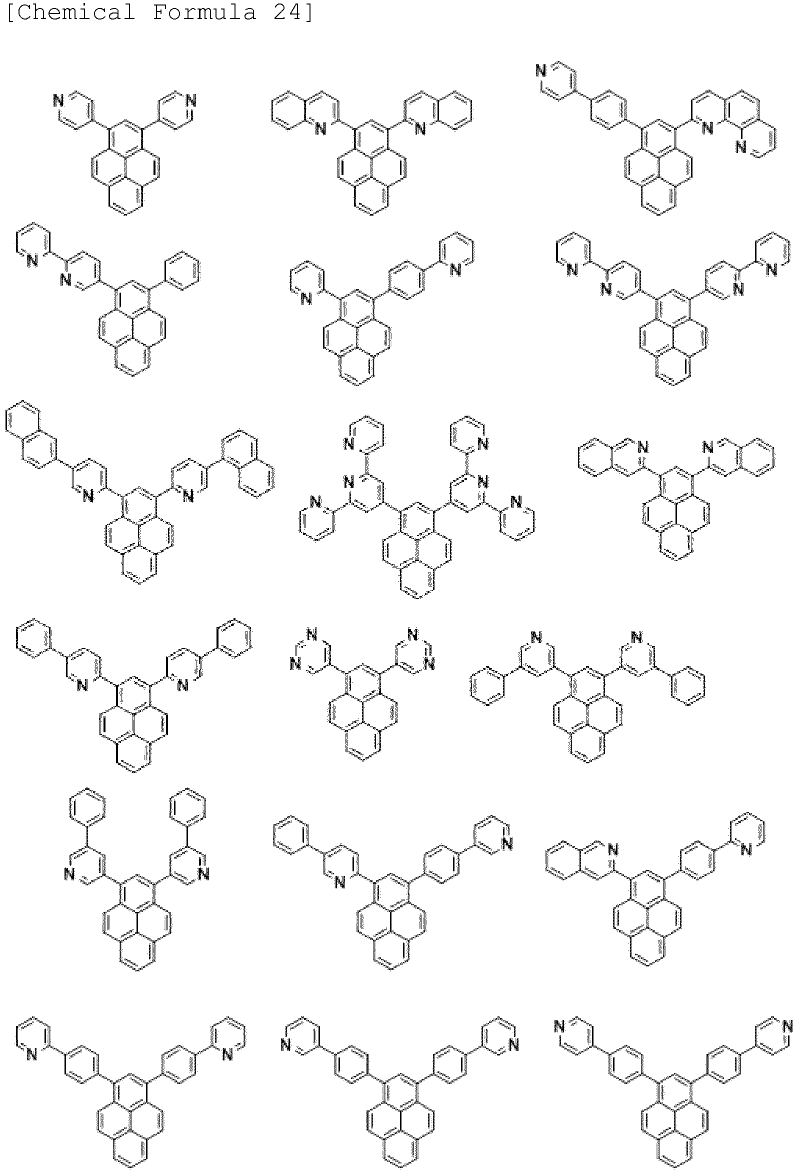

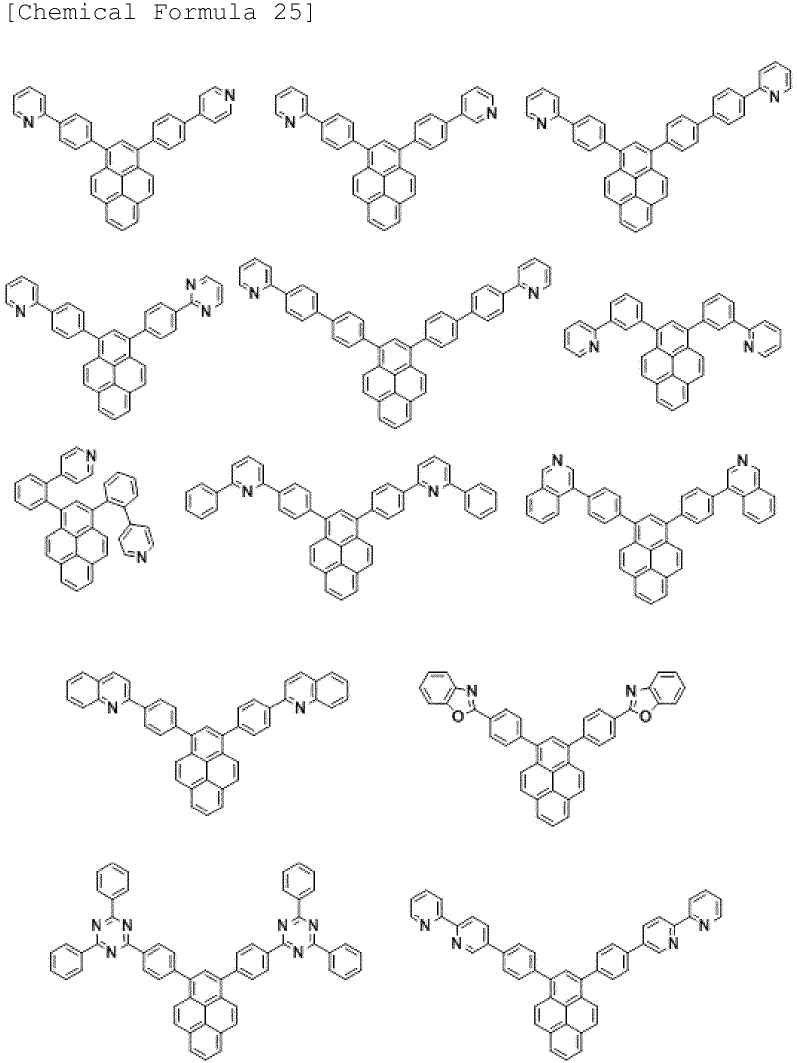

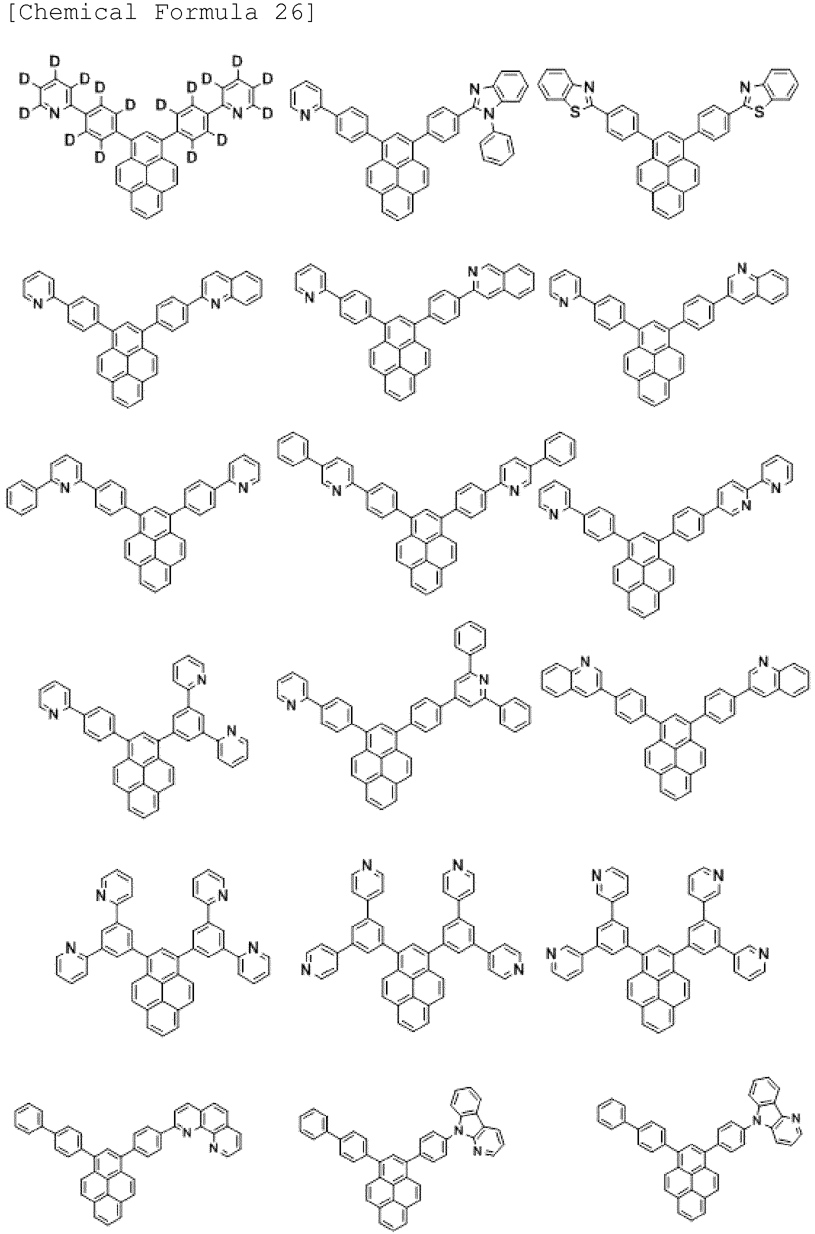

The pyrene compound represented by the general formula (1) is used as a light emitting device material. As used herein, the light emitting device material represents a material used in any layer of a light emitting device. As mentioned below, any layer of the light emitting device includes a protective film layer of a cathode, in addition to a layer selected from a hole transporting layer, an emissive layer, and an electron transporting layer. It is possible to obtain a light emitting device, which enables low driving voltage and is excellent in durability, by using the pyrene compound represented by the general formula (1) in any layer of the light emitting device.

The pyrene compound represented by the general formula (1) is preferably used in an emissive layer or an electron transporting layer of a light emitting device of the light emitting device since it has high electron injectability/transportability, luminance efficiency, and thin-film stability.

When the pyrene compound represented by the general formula (1) is used in the emissive layer, at least one of X¹ and X² is preferably an aryl group, or a heteroaryl group containing no electron-accepting nitrogen. It is necessary that the emissive layer transmits holes and electrons with good balance. In an embodiment not falling under the scope of the present invention, X¹ or X² is preferably an aryl group, or a heteroaryl group containing no electron-accepting nitrogen since hole transporting properties can be improved while making use of high electron transportability of a pyrene skeleton, and thus contributing to realize low voltage and high efficiency of the light emitting device. Both X¹ and X² are preferably aryl groups, or heteroaryl groups containing no electron-accepting nitrogen since hole transporting properties are improved. Both X¹ and X² are groups selected from an aryl group, a carbazolyl group, a dibenzofuranyl group, and a dibenzothiophenyl group since hole transporting properties are more improved.

According to the present invention, when at least one of X¹ and X² is an aromatic heterocyclic group containing electron-accepting nitrogen, the pyrene compound represented by the general formula (1) is preferably used in the electron transporting layer since it has excellent electron injectability/transportability.

Embodiments of the light emitting device of the present invention will be described in detail below. The light emitting device of the present invention includes an anode and a cathode, and an organic layer interposing between the anode and the cathode. The organic layer includes at least an emissive layer, and the emissive layer emits light from electric energy.

Examples of the lamination constitution of the organic layer include, in addition to the constitution composed only of an emissive layer, 1) hole transporting layer/emissive layer/electron transporting layer, 2) emissive layer/electron transporting layer, and 3) hole transporting layer/emissive layer. Each of the layers may be in the form of a single layer or a plurality of layers. When the hole transporting layer and the electron transporting layer have a plurality of layers, layers on sides contacting with an electrode are each referred to as a hole injection layer and an electron injection layer in some cases. However, in the following description, unless otherwise specified, a hole injection material is included in a hole transporting material, and an electron injection material is included in an electron transporting material, respectively.

In order to maintain the mechanical strength of the light emitting device, the light emitting device is preferably formed on a substrate. A glass substrate such as soda glass or alkali-free glass is suitably used as the substrate. Since it is favorable that the thickness of a glass substrate has a sufficient thickness for maintaining the mechanical strength, 0.5 mm or more is sufficient. Regarding the material of glass, since it is preferable that the amount of ions eluted from glass is low, alkali-free glass is preferable. Alternatively, since soda lime glass provided with a barrier coating such as SiO₂ is commercially available, it can also be used. It is not necessary that the substrate is glass and, for example, the anode may be formed on a plastic substrate.

In the light emitting device, the anode and the cathode have a role for supplying a sufficient current for light emission of the device. It is desirable that at least one of them is transparent or translucent in order to take out light from the light emitting device. Usually, the anode formed on a substrate is made to be a transparent electrode.

Examples of a material used in the anode includes, but are not particularly limited to, electrically conductive metal oxides such as tin oxide, indium oxide, indium tin oxide (ITO), and indium zinc oxide (IZO); metals such as gold, silver, and chromium; inorganic electrically conductive substances such as copper iodide and copper sulfide; and electrically conductive polymers such as polythiophene, polypyrrole, and polyaniline; as long as the material is a material which can efficiently inject holes into the organic layer, and is transparent or translucent so as to take out light. It is particularly desirable to use ITO glass or Nesa glass. These electrode materials may be used alone, or a plurality of materials may be used by lamination or mixing. Since it is favorable that a sufficient current for light emission of the device can be supplied, the resistance of a transparent electrode is not limited. From the viewpoint of the power consumption of the device, low resistance is desirable. For example, an ITO substrate having 300 Ω/□ or less functions as a device electrode. Since currently, it has become possible to supply a current to a substrate having about 10 Ω/□ it is particularly desirable to use a substrate having low resistance of 20 Ω/□ or less. The thickness of the anode can be arbitrarily selected according to the resistance value, and is usually used in a thickness between 100 to 300 nm in many cases.

The material used in the cathode is not particularly limited, as long as it is a substance which can efficiently inject electrons into the emissive layer. In general, the material is preferably metals such as platinum, gold, silver, copper, iron, tin, aluminum, and indium; alloys or multilayer laminates of these metals with metals having a low work function, such as lithium, sodium, potassium, calcium, and magnesium. Among them, metal selected from aluminum, silver, and magnesium is preferable in view of electric resistance value, ease of film formation, stability of a film, and luminance efficiency. It is particularly preferred that the cathode is composed of magnesium and silver since electron injection into the electron transporting layer and the electron injection layer becomes easy, and thus enabling low-voltage driving.

For example, it is preferred to laminate metals such as platinum, gold, silver, copper, iron, tin, aluminum, and indium; alloys using these metals; inorganic substances such as silica, titania, and silicon nitride; and organic polymer compounds such as polyvinyl alcohol, polyvinyl chloride, and hydrocarbon-based polymer compound; on the cathode as a protective film layer. The compound represented by the general formula (1) can also be utilized as this protective film layer. However, in the case of a device structure for taking out light from the cathode side (top emission structure), the material of the protective film layer is selected from materials having light permeability in a visible light region.

Examples of the method for producing these electrodes include, but are not particularly limited to, resistance heating, electron beam, sputtering, ion plating, and coating.

It is necessary that the hole transporting material efficiently transports holes from the anode between electrodes to which the electric field is given. Therefore, it is desirable that the hole transporting material exhibits high hole injection efficiency and efficiently transports injected holes. For this reason, it is required that the hole transporting material is a substance which has suitable ionization potential and enables large hole mobility, and is also excellent in stability and is less likely to generate impurities that become a trap during production and use. Preferable examples of substances satisfying such conditions include, but are not particularly limited to, triphenylamine derivatives such as 4,4'-bis(N-(3-methylphenyl)-N-phenylamino)biphenyl, 4,4'-bis(N-(1-naphthyl)-N-phenylamino)biphenyl, and 4,4',4"-tris(3-methylphenyl(phenyl)amino)triphenylamine; biscarbazole derivatives such as bis(N-allylcarbazole) or bis(N-alkylcarbazole); pyrazoline derivatives; stilbene-based compounds; hydrazone-based compounds; heterocyclic compounds such as benzofuran derivatives and thiophene derivatives, oxadiazole derivatives, phthalocyanine derivatives, and porphyrin derivatives; fullerene derivatives; polymer-based derivatives such as polycarbonate and styrene derivatives having the above-mentioned monomers on a side chain; polythiophene, polyaniline, polyfluorene, polyvinylcarbazole, and polysilane.

It is also possible to use inorganic compounds such as p-type Si and p-type SiC. It is also possible to use a compound represented by the following general formula (4), tetrafluorotetracyanoquinodimethane (4F-TCNQ) or molybdenum oxide. 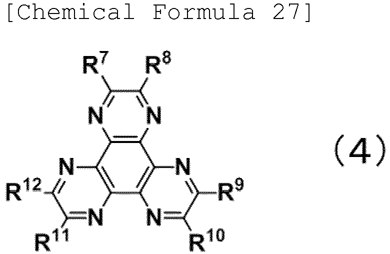 wherein R⁷ to R¹² may be the same or different, and are each selected from the group consisting of halogen, a sulfonyl group, a carbonyl group, a nitro group, a cyano group, and a trifluoromethyl group.

It is preferred that a compound (5) (1,4,5,8,9,12-hexaazatriphenylenehexacarbonitrile) is contained in a hole transporting layer or a hole injection layer since low-voltage driving is realized.

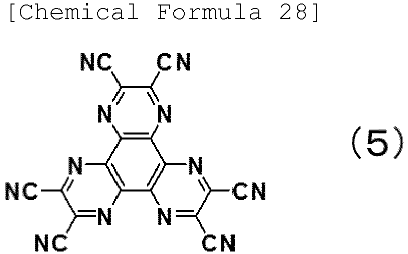

The hole transporting layer may be either formed of only one kind of a hole transporting material, or formed by laminating or mixing one or more kinds of hole transporting materials. Using a mixture of a hole transporting material and a polymer binder, the hole transporting layer may be formed. An inorganic salt such as iron(III) chloride may be added to the hole transporting material to form a hole transporting layer.

The emissive layer may be in the form of either a single layer or a plurality of layers. The emissive material may be either a mixture of the host material and the dopant material, or the host material alone. In the emissive layer, only the host material or the dopant material may emit light, or both of the host material and the dopant material emit light. From the viewpoint of efficiently utilizing electric energy and obtaining light emission at high color purity, the emissive layer is preferably composed of a mixture of the host material and the dopant material. The host material and the dopant material may be one kind or a combination of a plurality of kinds, respectively. The dopant material may be contained in a whole host material, or may be partially contained therein. The dopant material may be either laminated with a layer made of the host material, or dispersed in the host material. The dopant material can control a luminescent color by mixing the host material with the dopant material. Since when the amount of the dopant material is too large, concentration quenching occurs, it is used preferably in an amount of 20% by weight or less, and more preferably 10% by weight or less, based on the host material. As a doping method, the dopant material can be codeposited with the host material, or the dopant material may be mixed with the host material in advance, followed by deposition.

Specific examples of the emissive material include, but are not particularly limited to, fused ring derivatives such as anthracene and pyrene; metal chelated oxynoid compounds including tris(8-quinolinolate)aluminum; bisstyryl derivatives such as bisstyrylanthracene derivatives and distyrylbenzene derivatives; tetrapthenylbutadiene derivatives; indene derivatives, coumarine derivatives, oxadiazole derivatives, pyrrolopyridine derivatives, perinone derivatives, cyclopentadiene derivatives, oxadiazole derivatives, thiadiazolopyridine derivatives, dibenzofuran derivatives, carbazole derivatives, and indolocarbazole derivatives; and polymer-based derivatives such as polyphenylenevinylene derivatives, polyparaphenylene derivatives, and polythiophene derivatives.

Since the compound represented by the general formula (1) has high light emitting ability, it is suitably used as an emissive material. Since the compound represented by the general formula (1) exhibits strong light emission in an ultraviolet to blue region (300 to 450 nm region), it can be suitably used as a blue emissive material. Although the compound represented by the general formula (1) may be used as a dopant material, it is suitably used as a host material because of its excellent thin-film stability.

It is not necessary that the host material is limited to only one kind of the compound and a plurality of compounds may be used by mixing them. Examples of the host material include, but are not particularly limited to, compounds having a fused aryl ring such as naphthalene, anthracene, phenanthrene, pyrene, chrysene, naphthacene, triphenylene, perylene, fluoranthene, fluorene, and indene, and derivatives thereof; aromatic amine derivatives such as N,N'-dinaphthyl-N,N'-diphenyl-4,4'-diphenyl-1,1'-diamine; metal chelated oxynoid compounds including tris(8-quinolinato)aluminum (III); bisstyryl derivatives such as distyrylbenzene derivatives; tetraphenylbutadiene derivatives, indene derivatives, coumarine derivatives, oxadiazole derivatives, pyrrolopyridine derivatives, perinone derivatives, cyclopentadiene derivatives, pyrrolopyrrole derivatives, thiadiazolopyridine derivatives, dibenzofuran derivatives, carbazole derivatives, indolocarbazole derivatives, and triazine derivatives; and polymer-based derivatives such as polyphenylenevinylene derivatives, polyparaphenylene derivatives, polyfluorene derivatives, polyvinylcarbazole derivatives, and polythiophene derivatives. Among them, as a host which is used when the emissive layer performs phosphorescence emission, metal chelated oxynoid compounds, dibenzofuran derivatives, carbazole derivatives, indolocarbazole derivatives, triazine derivatives, and the like are suitably used.

Examples of the dopant material include, but are not particularly limited to, compounds having a fused aryl ring such as naphthalene, anthracene, phenanthrene, pyrene, chrysene, triphenylene, perylene, fluoranthene, fluorene, and indene, and derivatives thereof (for example, 2-(benzothiazol-2-yl)-9,10-diphenylanthracene and 5,6,11,12-tetraphenylnaphthacene); compounds having a heteroaryl ring such as furan, pyrrole, thiophene, silole, 9-silafluorene, 9,9'-spirobisilafluorene, benzothiophene, benzofuran, indole, dibenzothiophene, dibenzofuran, imidazopyridine, phenanthroline, pyridine, pyrazine, naphthyridine, quinoxaline, pyrrolopyridine, and thioxanthene, and derivatives thereof; borane derivatives; distyrylbenzene derivatives; aminostyryl derivatives such as 4,4'-bis(2-(4-diphenylaminophenyl)ethenyl)biphenyl, 4,4'-bis(N-(stilben-4-yl)-N-phenylamino)stilbene; aromatic acetylene derivatives; tetraphenylbutadiene derivatives; stilbene derivatives; aldazine derivatives; pyrromethene derivatives; diketopyrrolo[3,4-c]pyrrole derivatives; coumarine derivatives such as 2,3,5,6-1H,4H-tetrahydro-9-(2'-benzothiazolyl)quinolizino[9,9a,1-gh]coumarine; azole derivatives such as imidazole, thiazole, thiadiazole, carbazole, oxazole, oxadiazole, and triazole, and metal complexes thereof; and aromatic amine derivatives, a representative of which is N,N'-diphenyl-N,N'-di(3-methylphenyl)-4,4'-diphenyl-1,1'-diamine.

The dopant material used when the emissive layer performs phosphorescence emission is preferably a metal complex compound containing at least one metal selected from the group consisting of iridium (Ir), ruthenium (Ru), palladium (Pd), platinum (Pt), osmium (Os), and rhenium (Re). The ligand preferably has a nitrogen-containing aromatic heterocyclic ring such as a phenylpyridine skeleton or a phenylquinoline skeleton. However, the complex is not limited thereto, and a suitable complex is selected in context with luminescent color, device performance and host compound to be required.

The electron transporting layer is a layer in which electrons are injected from the cathode and, further, which transports the electrons. The electron transporting layer is required to have high electron injection efficiency and efficiently transport injected electrons. Therefore, the electron transporting layer is preferably composed of a substance that has large electron affinity, high electron mobility and excellent stability and is less likely to generate, during production and use, impurities which will act as a trap. Particularly, when layers are laminated in a large thickness, since a low-molecular compound is likely to be crystallized to cause deterioration of film quality, a compound having a molecular weight of 400 or more is preferably used so as to maintain stable film quality. However, considering transportation balance between holes and electrons, if the electron transporting layer mainly plays a role of efficiently inhibiting holes from flowing toward the cathode from the anode without being recombined, there is exerted the same effect of improving luminance efficiency as that in the case where the electron transporting layer is made of a material having a high electron transportation capability even if the electron transporting layer is made of a material having not so high an electron transportation capability.

The electron transporting material used in the present invention is not necessarily limited to only one kind of the compound, and a plurality of compounds may be used by mixing them. Examples of the electron transporting material include, but are not particularly limited to, compounds having a fused aryl ring such as naphthalene, anthracene, and pyrene, and derivatives thereof; styryl-based aromatic ring derivatives, a representative of which is 4,4'-bis(diphenylethenyl)biphenyl; perylene derivatives; perinone derivatives; coumarine derivatives; naphthalimide derivatives; quinone derivatives such as anthraquinone and diphenoquinone; phosphorus oxide derivatives; carbazole derivatives and indole derivatives; quinolinol complexes such as tris(8-quinolinolato)aluminum (III); hydroxyazole complexes such as hydroxyphenyloxazole complexes; azomethine complexes; tropolone metal complexes; and flavonol metal complexes.

The compound represented by the general formula (1) is used as an electron transporting material, particularly preferably, since it has high electron injectability/transportability. In case the electron transporting layer further contains the donor compound, the compound has high compatibility with the donor compound in a thin film state, and exhibits higher electron injectability/transportability. This mixture layer accelerates transportation of electrons to the emissive layer from the cathode, leading to a further improvement in effects of high luminance efficiency and low driving voltage.

The donor compound is a compound which makes easy electron injection into the electron transporting layer from the cathode or the electron injection layer and also improves electric conductivity of the electron transporting layer, by improving the electron injection barrier. Therefore, it is more preferable that the electron transporting layer contains the donor compound so as to improve electron transportability, in addition to the compound represented by the general formula (1).

Preferable examples of the donor compound include an alkali metal, an inorganic salt containing an alkali metal, a complex of an alkali metal and an organic substance, an alkali earth metal, an inorganic salt containing an alkali earth metal, or a complex of an alkali earth metal and an organic substance. Examples of preferable alkali metal and alkali earth metal include alkali metals such as lithium, sodium, and cesium; and alkali earth metals such as magnesium and calcium, which have a low work function and have significant effect of improving electron transportability.

Because of ease of deposition in vacuum and excellent handling, the donor compound is preferably in a state of an inorganic salt or a complex of metal and an organic substance, rather than a metal single substance. In view of easy handling in the atmospheric air, and easiness in control of the concentration to be added, the donor compound is more preferably in a state of a complex of an alkali metal and an organic substance, or a complex of an alkali earth metal and an organic substance. Examples of the inorganic salts include oxides such as LiO and Li₂O; nitrides; fluorides such as LiF, NaF, and KF; and carbonates such as Li₂CO₃, Na₂CO₃, K₂CO₃, Rb₂CO₃, and Cs₂CO₃. Preferable examples of the alkali metal or the alkali earth metal include lithium in view of low cost of raw materials and ease of synthesis. Preferable examples of the organic substance in complexes of metal and an organic substance include quinolinol, benzoquinolinol, flavonol, hydroxyimidazopyridine, hydroxybenzoazole, and hydroxytriazole. Among them, complexes of an alkali metal and an organic substance are preferable, complexes of lithium and an organic substance are more preferable, and lithium quinolinol is particularly preferable.

When the content of the donor compound in the electron transporting layer is suitable, the injection ratio of electrons into the electron transporting layer from the cathode or the electron injection layer is increased, and an energy barrier between the cathode and the electron injection layer, or between the electron injection layer and the electron transporting layer is reduced, and a driving voltage is reduced. Although suitable content of the donor compound varies depending on a material or the film thickness of a doping region, the electron transporting layer is preferably formed by deposition so that the deposition rate ratio of the organic compound and the donor compound falls within a range from 100:1 to 1:100. The deposition rate ratio is more preferably from 10:1 to 1:10, and particularly preferably from 7:3 to 3:7.

A method of doping the electron transporting layer with the donor compound to improve electron transporting ability exerts particularly the effect when the organic layer has a large thickness. The method is particularly preferably used when the total thickness of the electron transporting layer and the emissive layer is 50 nm or more. For example, there is a method of utilizing the interference effect for improving luminance efficiency, and this improves efficiency of taking out light by matching the phase of light which is directly radiated from the emissive layer, and the phase of light which is reflected at the cathode. This optimal condition varies depending on the emission wavelength of light, and it results in 50 nm or more of the total thickness of the electron transporting layer and the emissive layer, and in the case of long wavelength light emission such as a red color, the thickness becomes near 100 nm in some cases.

The film thickness of the electron transporting layer to be doped with the donor compound may be part or all of the electron transporting layer, and as the film thickness of the whole electron transporting layer is larger, the larger concentration of doping is better. When part is doped, it is desirable to provide a doping region on at least an electron transporting layer/cathode interface and, the effect of reducing a voltage is obtained even doping is carried out around the cathode interface. On the other hand, if the emissive layer is doped with the donor compound, it gives an adverse influence of reducing luminance efficiency, it is desirable to provide a non-doped region at an emissive layer/electron transporting layer interface.

Examples of the method of forming each layer constituting the light emitting device include, but are not limited to, a resistance heating evaporation method, an electron beam evaporation method, a sputtering method, a molecular stacking method, and a coating method. In view of device characteristics, a resistance heating evaporation method or an electron beam evaporation method is usually preferred.

Although the thickness of the organic layer depends on the resistance value of an emissive substance and cannot be limited, it is selected from between 1 nm and 1,000 nm. The thickness of each of the emissive layer, the electron transporting layer and the hole transporting layer is preferably 1 nm or more and 200 nm or less, and more preferably 5 nm or more and 100 nm or less.

The light emitting device has a function of successfully converting electric energy into light. While a DC current is mainly used as the electric energy, a pulse current or an AC current can also be used. The values of the electric current and the voltage are not particularly limited. However, taking into consideration the power consumption and the life of the device, the values are preferably selected so that a maximum luminance can be obtained at energy as low as possible.

The light emitting device of the present invention is used suitably as matrix and/or segment system displays.

In the matrix system, pixels for display are two-dimensionally disposed in lattice or mosaic, and characters and images are displayed by sets of pixels. The shape and size of the pixels are determined according to the intended application. In the case of image and character display by personal computers, monitors and televisions, there are normally used quadrangular pixels with up to 300 µm sides, and in the case of large-size displays such as display panels, there are normally used pixels with sides of the mm order. Pixels in the same color may be merely arrayed in the case of monochrome display, while pixels in red, green and blue are arrayed for indication in the case of color display. In a color display, the arrangement system typically includes a delta type system and a stripe type system. The method of driving the matrix may be either line-sequential driving or active matrix driving. While the line-sequential driving is simple in the structure of the display, active matrix driving is sometimes more advantageous when taking operation characteristics into consideration. Therefore, driving method is properly used according to the intended applications.

The segment system is a system wherein a pattern is formed so as to display prescribed information and the range determined by the arrangement of the pattern is caused to emit light. Examples of segment system displays include time and temperature displays in digital watches and thermometers, operation state displays in audio instruments and microwave cookers, and vehicle panel displays. The matrix display and the segment display may be present together in the same panel.

The light emitting device of the present invention can also be preferably employed as a backlight of various instruments. The backlight is mainly used for the purpose of improving visibility of a display device which itself emits no light, and it is used in liquid crystal display devices, watches, audio devices, automobile panels, display plates, and signs. The light emitting device of the present invention is preferably used as the backlight of a liquid crystal display device, particularly a personal computer, the thickness reduction of which is being studied. The light emitting device of the present invention can provide a backlight that is smaller in thickness and weight than conventional products.

### [Examples]

The present invention will be described by way of Examples, but the present invention is not limited to these Examples.

### Synthesis Example 1

### Synthesis of Compound [1]

A mixed solution of 150 g of pyrene, 75.52 g of t-butyl chloride, and 742 ml of dichloromethane was cooled to 0°C under a nitrogen gas flow, and 98.9 g aluminum chloride was added. After stirring this mixed solution at room temperature for 3 hours, 1,100 ml of water was poured into the solution, followed by extraction with 1,100 ml of dichloromethane. The obtained organic layer was washed three times with 750 ml of water, dried over magnesium sulfate and then evaporated. The obtained solid was purified by washing with methanol and filtered. The obtained solid was vacuum-dried to obtain 272 g of a brown solid containing 65% by weight of 2-t-butylpyrene.

Subsequently, a mixed solution of 6 g (content of 2-t-butylpyrene: 65%) of the brown solid, 100 ml of dichloromethane, and 30 ml of methanol was cooled to 0°C under a nitrogen gas flow, and 6.6 g of benzyltrimethylammonium tribromide dissolved in 20 ml of dichloromethane was added dropwise. After stirring this mixed solution at room temperature for 2 hours, 100 ml of water was poured into the solution, followed by extraction with 100 ml of dichloromethane. The obtained organic layer was washed twice with 100 ml of water, dried over magnesium sulfate and then filtered. The filtrate was evaporated and 20 ml of methanol was added to the obtained solid, followed by stirring for 10 minutes and further filtration. To the obtained solid, 60 ml of hexane was added, followed by stirring for 30 minutes and further filtration. The obtained solid was vacuum-dried to obtain a solid containing 4.6 g (79.2% by weight) of 1-bromo-7-t-butylpyrene.

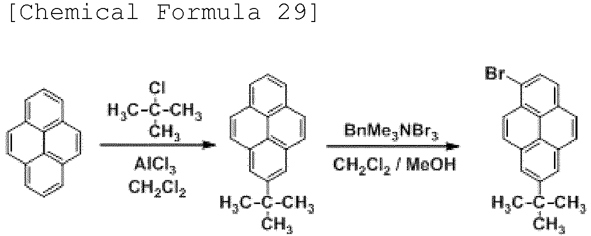

Subsequently, a mixed solution of 4.6 g (content: 79.2%) of a solid containing 1-bromo-7-t-butylpyrene, 1.9 g of 4-chlorophenylboric acid, 54 mL of 1,2-dimethoxyethane, and 16 mL of an aqueous 1.5M sodium carbonate solution was nitrogen-substituted and then 76 mg of bis(triphenylphosphine)palladium dichloride was added, followed by heating and drying at 60°C for 4 hours. The reaction mixture was cooled to room temperature and then extracted with 100 ml of toluene. The obtained organic layer was washed three times with 50 ml of water, dried over magnesium sulfate and then filtered. The filtrate was evaporated and then purified by silica gel column chromatography. After evaporating the eluate, 50 ml of methanol was added to the obtained solid, followed by filtration. The obtained solid was vacuum-dried to obtain 3.2 g (yield: 81%) of 7-t-butyl-1-(4-chlorophenyl)pyrene.

Subsequently, a mixed solution of 3.2 g of 7-t-butyl-1-(4-chlorophenyl)pyrene, 50 ml of dichloromethane, and 16 ml of methanol was cooled to 0°C under a nitrogen gas flow, and 3.9 g of benzyltrimethylammonium tribromide dissolved in 10 m of dichloromethane was added dropwise. This mixed solution was stirred at room temperature for 2 hours and 40 ml was poured into the solution, followed by extraction with 40 ml of dichloromethane. The obtained organic layer was washed twice with 40 ml of water, dried over magnesium sulfate and then evaporated. To the obtained solid, 20 ml of methanol was added and the mixture was left to stand overnight. The precipitated solid was filtered and vacuum-dried to obtain 4.3 g (yield: 97%) of an intermediate (A).

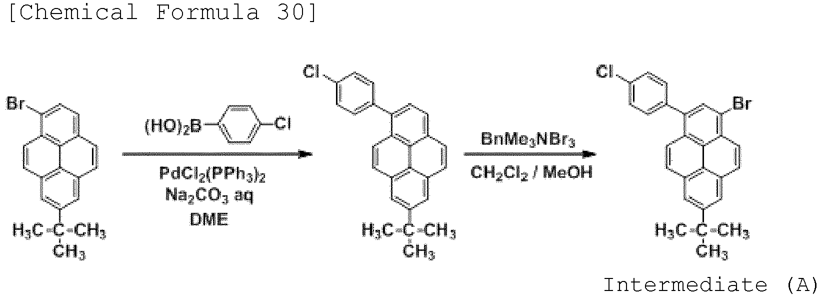

Subsequently, a mixed solution of 4.3 g of an intermediate (A), 2.1 g of 4-biphenylboric acid, 48 mL of 1,2-dimethoxyethane, and 14 mL of an aqueous 1.5M sodium carbonate solution was nitrogen-substituted and then 67 mg of bis(triphenylphosphine)palladium dichloride was added, followed by heating and stirring under reflux for 2 hours. The reaction mixture was cooled to room temperature and then extracted with 50 mL of toluene. The obtained organic layer was washed twice with 50 ml of water and then magnesium sulfate and activated carbon were added, followed by stirring at room temperature for 30 minutes and further filtration with celite. The filtrate was evaporated and then 8 ml of toluene was added to dissolve the obtained solid, and 20 ml of hexane was added dropwise to the solution. The precipitated solid was filtered and vacuum-dried to obtain 4.5 g (yield: 89%) of 1-(4-biphenyl)-7-t-butyl-3-(4-chlorophenyl)pyrene.

Subsequently, a mixed solution of 4.5 g of 1-(4-biphenyl)-7-t-butyl-3-(4-chlorophenyl)pyrene, 1.7 g of aluminum trichloride, and 170 ml of ortho-xylene was heated and stirred under a nitrogen gas flow at 60°C for 6 hours. The reaction mixture was cooled to room temperature and then 80 ml of water was poured into the solution, followed by extraction with 100 ml of toluene added. The obtained organic layer was washed twice with 100 ml of water, dried over magnesium sulfate and then filtered. The filtrate was evaporated and purified by silica gel column chromatography. The eluate was evaporated and then the obtained solid was heated and dissolved in 50 ml of toluene. To this solution, 50 ml of ethyl acetate was further added and stirred at room temperature for 5 hours, and then the precipitated solid was filtered. The solid was washed with methanol and vacuum-dried to obtain 2.5 g (yield: 63%) of an intermediate (B).

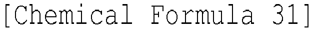

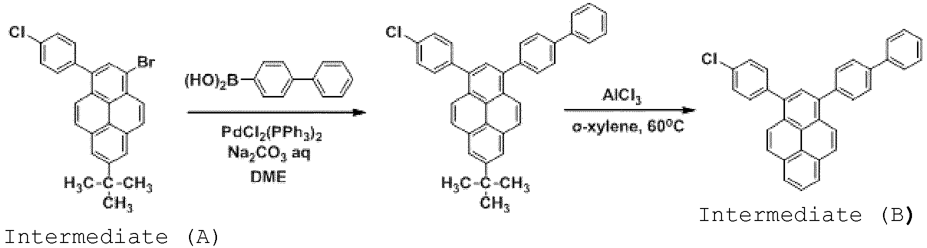

Subsequently, a mixed solution of 2.5 g of an intermediate (B), 0.99 g of 4-pyridineboric acid, 62 mg of bis(dibenzylidineacetone)palladium, 37 mg of tricyclohexylphosphine tetrafluoroborate, and 27 ml of 1,4-dioxane was nitrogen-substituted and then 7.2 ml of an aqueous 1.27M tripotassium phosphate solution was added, followed by heating and stirring under a nitrogen gas flow under reflux for 3 hours. The reaction mixture was cooled to room temperature and then 27 ml of water was added and the precipitate was filtered, and the obtained precipitate was dried by a vacuum dryer. The precipitate was purified by silica gel column chromatography and the eluate was evaporated, and then 20 ml of methanol was added to the obtained solid, followed by filtration. The obtained solid was vacuum-dried and purified by recrystallization with 80 mL of toluene to obtain 1.7 g (yield: 62%) of a yellow crystal.

¹H-NMR analytical results of the obtained yellow crystal are as follows, and revealed that the yellow crystal obtained above is the compound [1].
¹H-NMR (CDCl₃) δ 7.40(1H, t, J=3.8Hz), 7.48-7.56 (2H, m), 7.65(2H, dd, J=1.6Hz, 5.9Hz), 7.70-7.89(10H, m), 8.01-8.12(4H, m), 8.22(2H, d, J=9.7Hz), 8.28(2H, dd, J=10.8Hz, 17.0Hz), 8.73(2H, dd, J=1.6Hz, 6.5Hz).

This compound [1] was used as a light emitting device material after subjecting to sublimation refining under a pressure of 1 × 10⁻³ Pa at about 300°C, using an oil diffusion pump. HPLC purity (area % at a measuring wavelength of 254 nm) of this compound [1] was 99.9% before sublimation refining, and 99.9% after sublimation refining.

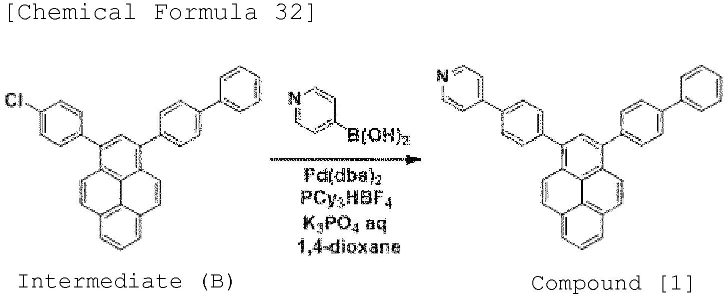

### Synthesis Example 2

### Synthesis of Compound [2]

A mixed solution of 10.0 g of an intermediate (A), 7.1 g of 3-(9-carbazolyl)phenylboric acid, 111 mL of 1,2-dimethoxyethane, and 33 mL of an aqueous 1.5M sodium carbonate solution was nitrogen-substituted and then 157 mg of bis(triphenylphosphine)palladium dichloride was added, followed by heating and stirring under reflux for 2 hours. The reaction mixture was cooled to room temperature and then extracted with 100 mL of toluene. The obtained organic layer was washed twice with 100 ml of water, dried over magnesium sulfate and then filtered. The filtrate was evaporated and purified by silica gel column chromatography. The eluate was evaporated 100 ml of methane was added to the obtained solid, followed by filtration. The obtained solid was vacuum-dried to obtain 11.5 g (yield: 85%) of 7-t-butyl-1-(3-(9-carbazolylphenyl))-3-(4-chlorophenyl)pyrene.

Subsequently, a mixed solution of 6.0 g of 7-t-butyl-1-(3-(9-carbazolylphenyl))-3-(4-chlorophenyl)pyrene, 1.6 g of aluminum trichloride, and 197 ml of ortho-xylene was heated and stirred under a nitrogen gas flow at 60°C for 4 hours. After cooling the reaction mixture to room temperature, 200 ml of water was poured into the solution, followed by extraction with 100 ml of toluene added. The obtained organic layer was washed twice with 200 ml of water, dried over magnesium sulfate and then filtered. The filtrate was evaporated and the obtained solid was purified by silica gel column chromatography. The eluate was evaporated and then 100 ml of methanol was added to the obtained solid, followed by filtration. The obtained solid was vacuum-dried to obtain 3.8 g (yield: 70%) of 1-(3-(9-carbazolylphenyl))-3-(4-chlorophenyl)pyrene.

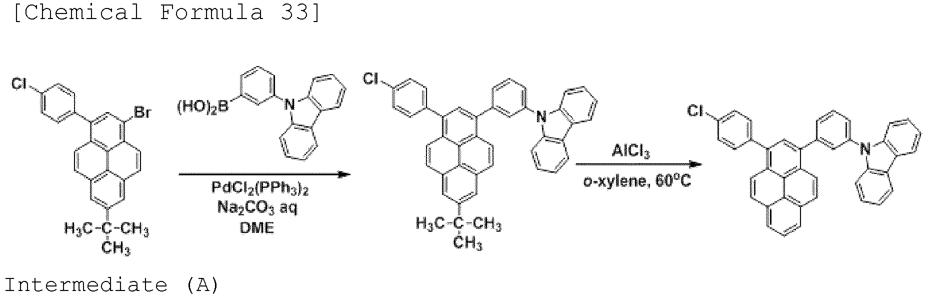

Subsequently, a mixed solution of 3.8 g of 1-(3-(9-carbazolylphenyl))-3-(4-chlorophenyl)pyrene, 1.3 g of 4-pyridineboric acid, 79 mg of bis(dibenzylidineacetone)palladium, 48 mg of tricyclohexylphosphine tetrafluoroborate, and 34 ml of 1,4-dioxane was nitrogen-substituted and 9.2 ml of an aqueous 1.27M tripotassium phosphophate solution was added, followed by heating and stirring under a nitrogen gas flow under reflux for 6 hours. After cooling the reaction mixture to room temperature, 34 ml of water was added, followed by extraction with 34 ml of toluene. The obtained organic layer was washed twice with 50 ml of water, dried over magnesium sulfate and then filtered. The filtrate was evaporated and purified by silica gel column chromatography. The eluate was evaporated and then the obtained solid was purified by recrystallization with 150 mL of toluene. The obtained solid was purified again by recrystallization with 120 ml of toluene, and then vacuum-dried to obtain 1.8 g (yield: 44%) of a yellow crystal.

¹H-NMR analytical results of the obtained yellow crystal are as follows, and revealed that the yellow crystal obtained above is the compound [2].
¹H-NMR (CDCl₃) δ 7.30(2H, dt, J=0.84Hz, 7.3Hz), 7.44(2H, dt, J=1.4Hz, 7.0Hz), 7.55-7.64(4H, m), 7.68-7.93(8H, m), 8.04-8.26(9H, m), 8.35(1H, d, J=9.2Hz), 8.72(2H, dd, J=4.1Hz, 1.6Hz).

This compound [2] was used as a light emitting device material after subjecting to sublimation refining under a pressure of 1 × 10⁻³ Pa at about 300°C, using an oil diffusion pump. HPLC purity (area % at a measuring wavelength of 254 nm) of this compound [2] was 99.7% before sublimation refining, and 99.8% after sublimation refining.

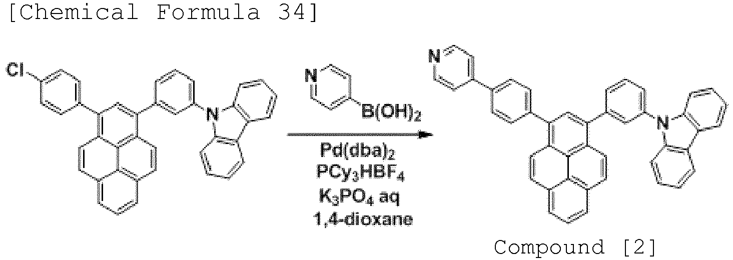

### Synthesis Example 3

### Synthesis of Compound [3]

A mixed solution of 2.8 g of an intermediate (B), 1.3 g of 1-naphthaleneboric acid, 3.8 g of tripotassium phosphate, and 30 ml of toluene was nitrogen-substituted and then 172 mg of bis(dibenzylidineacetone)palladium and 208 mg of tri-t-butylphosphine tetrafluoroborate were added, followed by heating and stirring under a nitrogen gas flow under reflux for 4 hours. After cooling the reaction mixture to room temperature, 30 ml of water was added, followed by extraction with 34 ml of toluene. The obtained organic layer was washed twice with 30 ml of water, dried over magnesium sulfate and then filtered. The filtrate was evaporated and purified by silica gel column chromatography. The eluate was evaporated and then the obtained solid was purified by recrystallization with a mixed solution of 27 ml of heptane and 27 ml of toluene. The obtained solid was purified again by recrystallization with a mixed solution of 27 ml of heptane and 27 ml of toluene, and then vacuum-dried to obtain 1.8 g (yield: 54%) of a yellow crystal.

¹H-NMR analytical results of the obtained yellow crystal are as follows, and revealed that the yellow crystal obtained above is the compound [3].
¹H-NMR (CDCl₃) : 7.41(1H,tt, J=7.6Hz, 0.81Hz), 7.48-7.64(6H, m), 7.68-7.86(10H, m), 7.88-7.98(2H, m), 8.02-8.17(5H, m), 8.18-8.24(2H, m), 8.33(1H, d, J=9.2Hz), 8.40(1H, d, J=9.5Hz).

This compound [3] was used as a light emitting device material after subjecting to sublimation refining under a pressure of 1 × 10⁻³ Pa at about 280°C, using an oil diffusion pump. HPLC purity (area % at a measuring wavelength of 254 nm) of this compound [3] was 99.7% before sublimation refining, and 99.8% after sublimation refining.

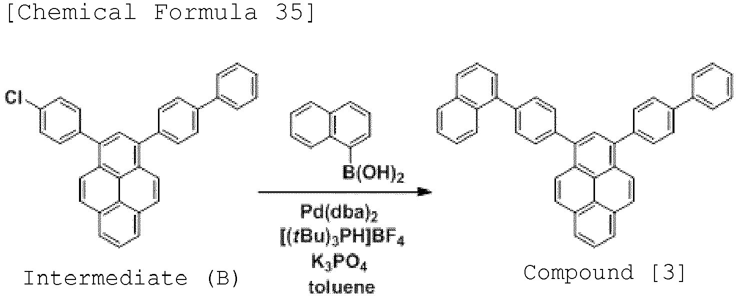

### Example 1

A glass substrate (manufactured by GEOMATEC Co., Ltd., 11 Ω/□, sputtered product), on which an ITO transparent electrically conductive film had been deposited in a thickness of 165 nm, was cut into a size of 38 × 46 mm, followed by subjecting to etching, and thus forming the ITO transparent electrically conductive film into prescribed electrode shape. The resulting substrate was ultrasound-washed with "SEMICOCLEAN 56" (trade name, manufactured by Furuuchi Chemical Corporation) for 15 minutes, and then washed with ultrapure water. This substrate was treated with UV-ozone for 1 hour immediately before producing a device, and placed in a vacuum evaporation equipment, followed by evacuation until the degree of vacuum in the equipment became 5 × 10⁻⁴ Pa or less. On the ITO transparent electrically conductive film, using a resistance heating method, first, 1,4,5,8,9,12-hexaazatriphenylenehexacarbonitrile as a material of a hole injection layer was deposited in a thickness of 5 nm and 4,4'-bis(N-(1-naphthyl)-N-phenylamino)biphenyl as a material of a hole transporting layer was deposited in a thickness of 60 nm, respectively. Then, the compound (H-1) as a host material and the compound (D-1) as a dopant material were deposited as materials of an emissive layer in a thickness of 40 nm so that the doping concentration became 5% by weight. Then, a mixture of the compound [1] and lithium fluoride as a donor compound was deposited as a material of an electron transporting layer in a thickness of 25 nm at a ratio of a deposition rate of 1:1 (0.05 nm/s:0.05 nm/s).

Subsequently, lithium fluoride was deposited in a thickness of 0.5 nm, and then aluminum was deposited in a thickness of 1,000 nm to obtain a cathode, and a device having a light emitting surface measuring 5 × 5 mm square was produced. As used herein, the film thickness is a value displayed by a quartz oscillation-type film thickness monitor. This light emitting device was direct-current driven at 10 mA/cm². As a result, the obtained light emitting device enables low driving voltage and high-efficiency light emission, and is also excellent in durability, and exhibited a driving voltage of 4.6 V, an external quantum efficiency of 5.3%, and luminance half-life of 6,500 hours.

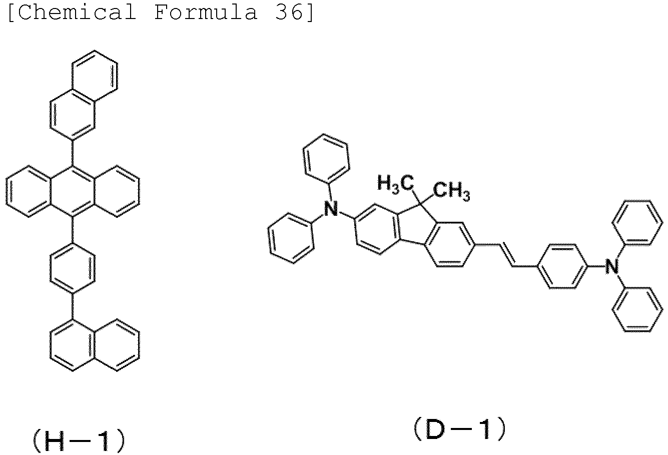

### Examples 2 to 4, 7, and 8, reference Examples 5, 6, and 9

In the same manner as in Example 1, expect that each of materials shown in Table 1 was used as a material of an electron transporting layer, light emitting devices were produced. The results are shown in Table 1. In Table 1, (2E-1) is the following compound.

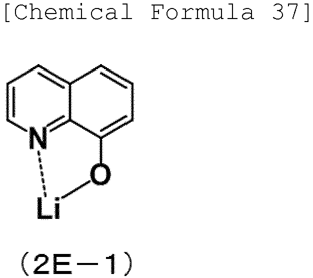

### Comparative Examples 1 to 6

In the same manner as in Example 1, expect that each of materials shown in Table 1 was used as a material of an electron transporting layer, light emitting devices were produced. The results are shown in Table 1. In Table 1, (E-1) and (E-2) are the following compounds.

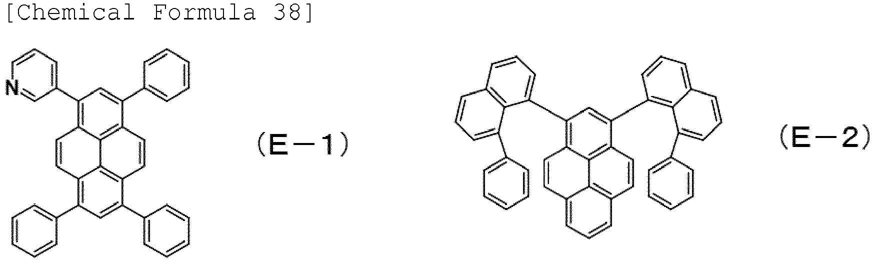

**[Table 1]**

| | Emissive material | | | Electron transporting layer | | Anode | External quantum efficiency (%) | Driving voltage (V) | Luminance half-life (h) |
|---|---|---|---|---|---|---|---|---|---|
| | Host material | Dopant material | Luminescent color | Compound | Donor compound | Metal | | | |
| Example 1 | H-1 | D-1 | Blue | Compound [1] | Lithium fluoride | Al | 5.3 | 4.6 | 6,500 |
| Example 2 | | | Blue | Compound [1] | None | Al | 4.1 | 5.2 | 5,000 |
| Example 3 | | | Blue | Compound [2] | Lithium fluoride | Al | 5.4 | 4.7 | 6,400 |
| Example 4 | | | Blue | Compound [2] | None | Al | 4.2 | 5.2 | 5,100 |
| Reference Example 5 | | | Blue | Compound [3] | Lithium fluoride | Al | 5.2 | 4.8 | 6,700 |
| Reference Example 6 | | | Blue | Compound [3] | None | Al | 4.3 | 5.3 | 5,300 |
| Example 7 | | | Blue | Compound [1] | 2E-1 | Al | 5.9 | 4.1 | 7,300 |
| Example 8 | | | Blue | Compound [2] | 2E-1 | Al | 5.7 | 4.3 | 7,400 |
| Reference Example 9 | | | Blue | Compound [3] | 2E-1 | Al | 5.8 | 4.0 | 7,500 |
| Comparative Example 1 | | | Blue | E-1 | Lithium fluoride | Al | 3.8 | 6.9 | 4,100 |
| Comparative Example 2 | | | Blue | E-1 | 2E-1 | Al | 4.2 | 6.2 | 4,600 |
| Comparative Example 3 | | | Blue | E-1 | None | Al | 2.8 | 7.3 | 3,000 |
| Comparative Example 4 | | | Blue | E-2 | Lithium fluoride | Al | 3.6 | 7.1 | 3,800 |
| Comparative Example 5 | | | Blue | E-2 | 2E-1 | Al | 4.3 | 6.5 | 4,200 |
| Comparative Example 6 | | | Blue | E-2 | None | Al | 2.9 | 7.9 | 2,600 |

### Example 10

In the same manner as in Example 1, except that a mixture of the compound [1] and a donor compound (2E-1) as a material of an electron transporting layer was deposited in a thickness of 25 nm at a deposition rate ratio 1:1 (0.05 nm/s:0.05 nm/s) and (2E-1) was deposited in a thickness of 0.5 nm, and then magnesium and silver as a cathode were codeposited in a thickness of 15 nm at a ratio of a deposition rate (magnesium:silver) of 10:1 (0.05 nm/s:0.05 nm/s), a light emitting device was produced.

This light emitting device was direct-current driven at 10 mA/cm². As a result, the obtained light emitting device enables low driving voltage and high-efficiency light emission, and is also excellent in durability, and exhibited a driving voltage of 3.7 V, an external quantum efficiency of 5.9%, and luminance half-life of 7,600 hours.

### Reference Examples 12 to 34 Examples 11 and 35 to 60

In the same manner as in Example 10, expect that each of materials shown in Table 2 and Table 3 was used as a material of an electron transporting layer, light emitting devices were produced. The results are shown in Table 2 and Table 3. In Table 2, the compounds [4] to [51] are the following compounds.

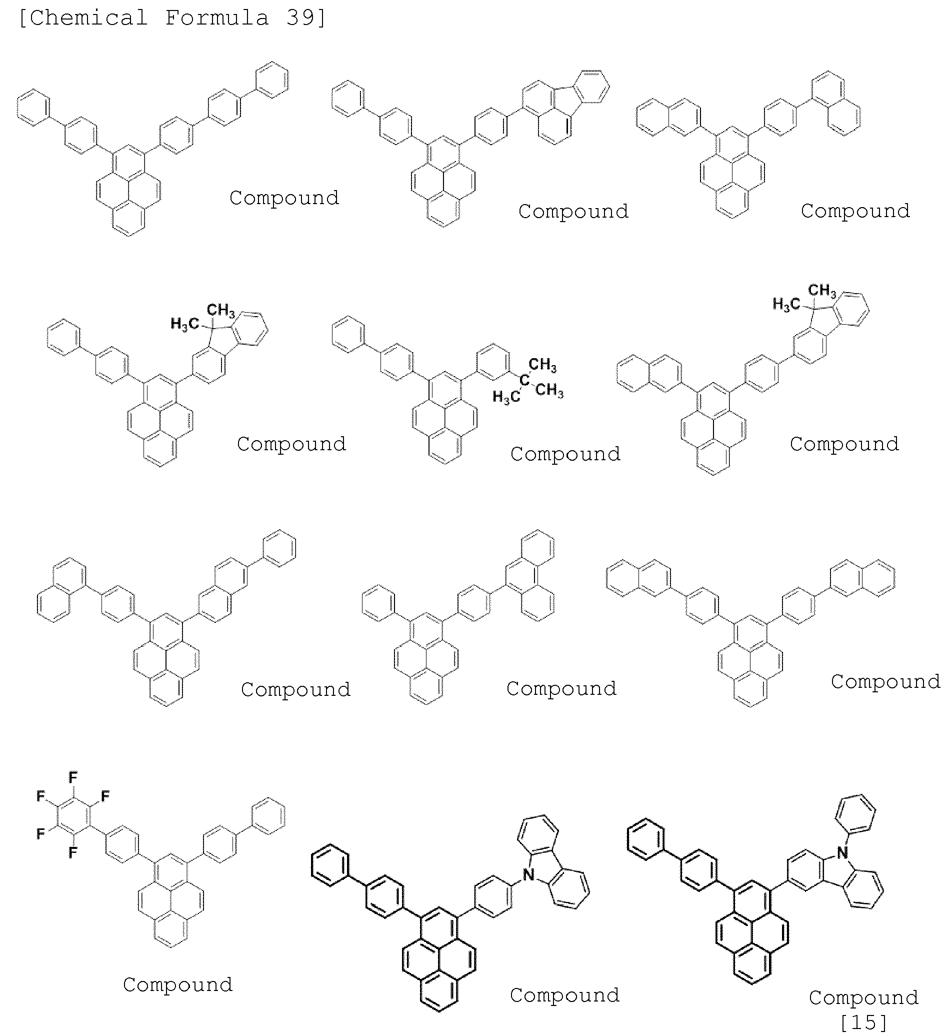

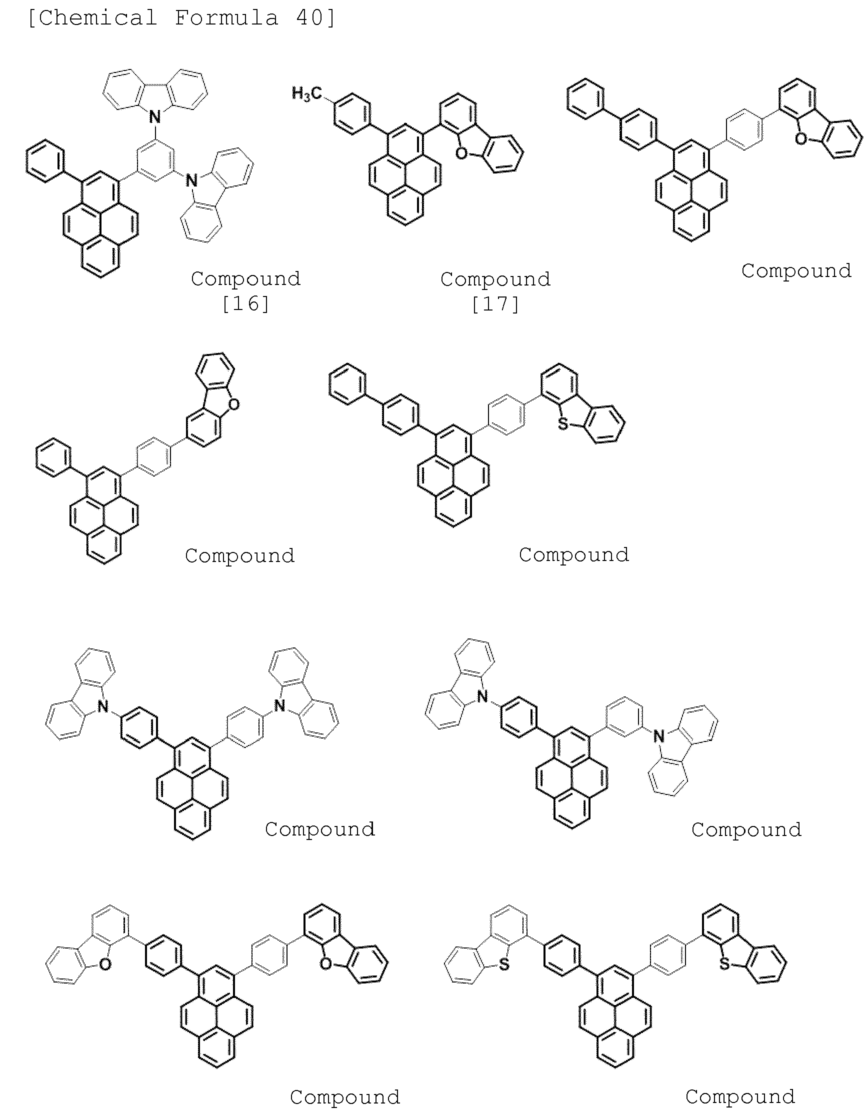

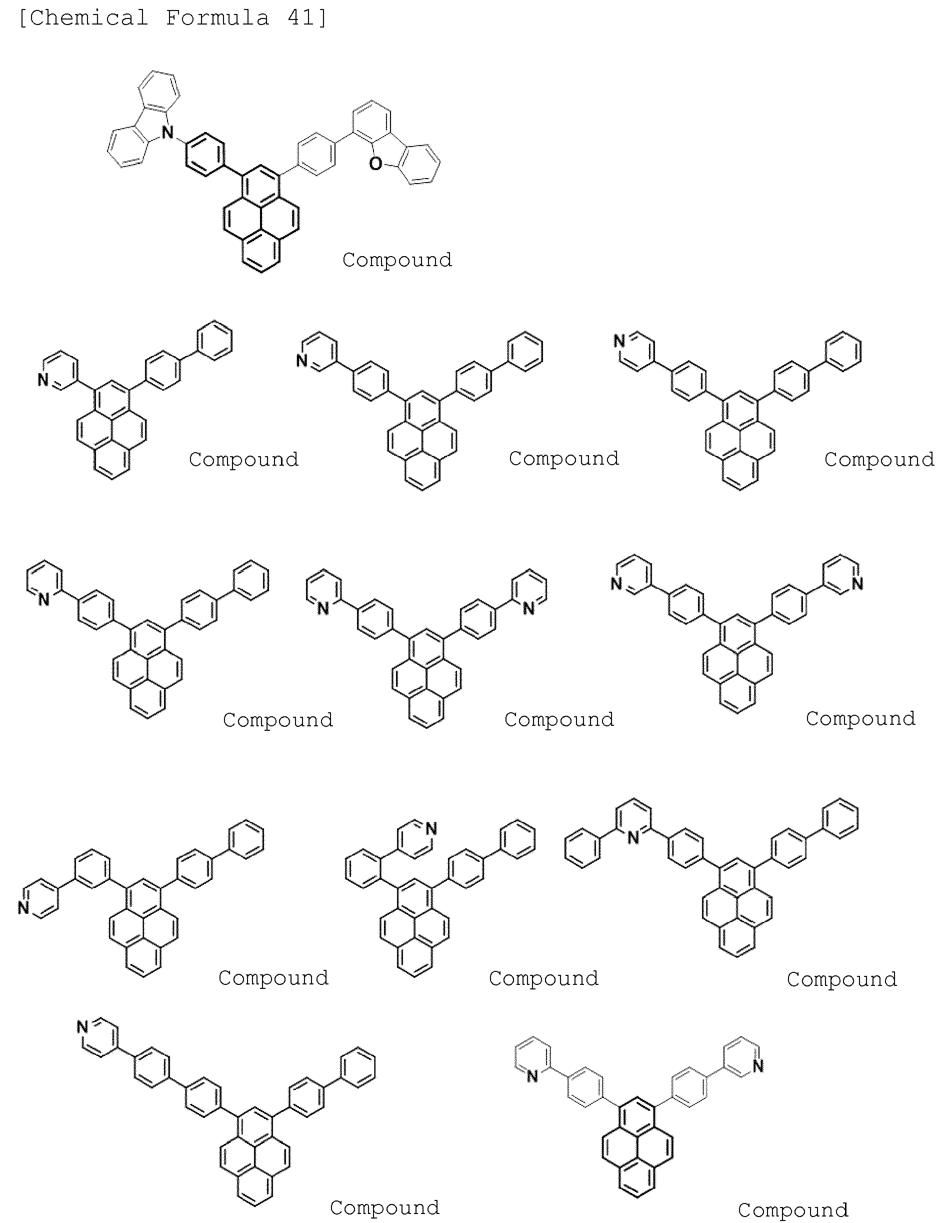

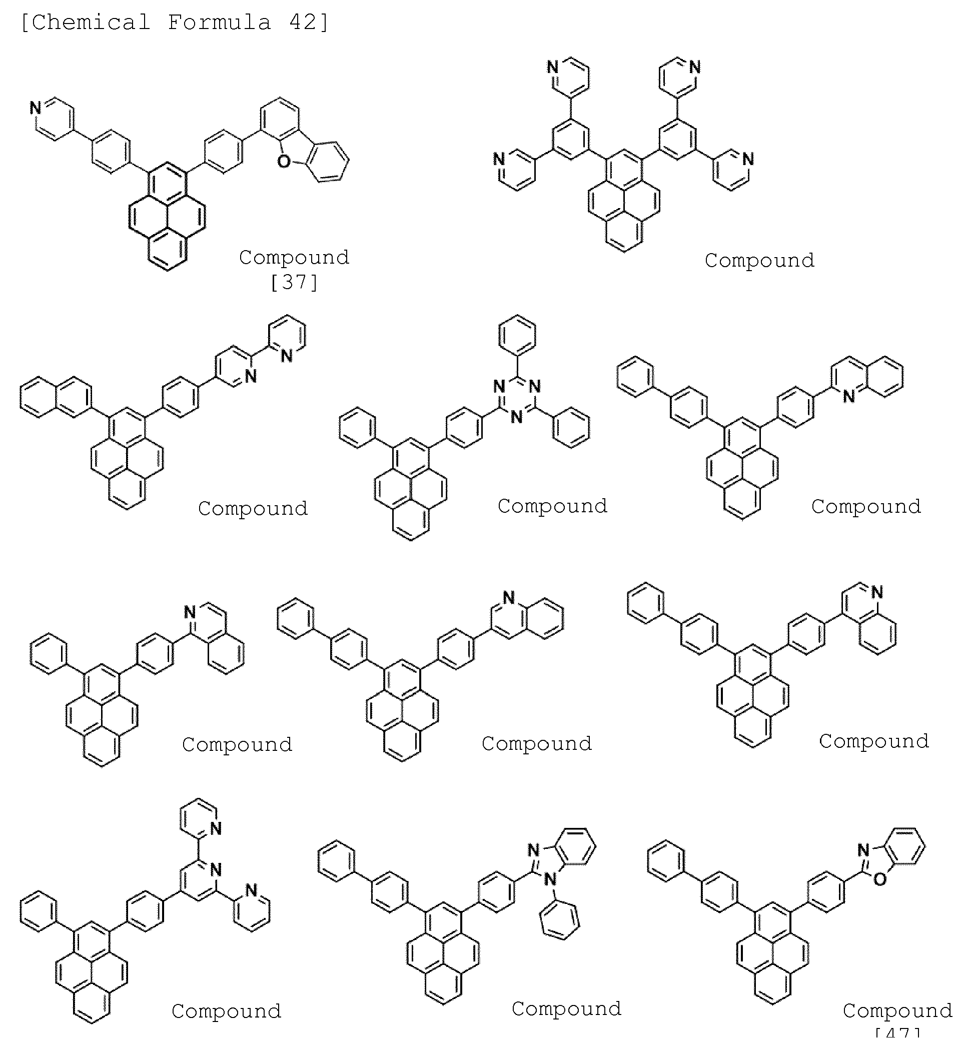

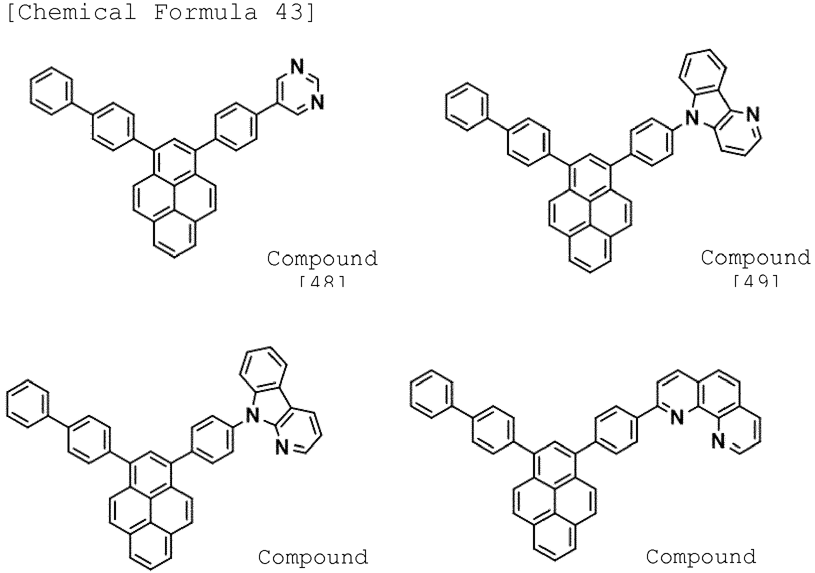

### Comparative Examples 7 to 10

In the same manner as in Example 10, expect that each of materials shown in Table 3 was used as a material of an electron transporting layer, light emitting devices were produced. The results are shown in Table 3. In Table 3, (E-3) and (E-4) are the following compound.

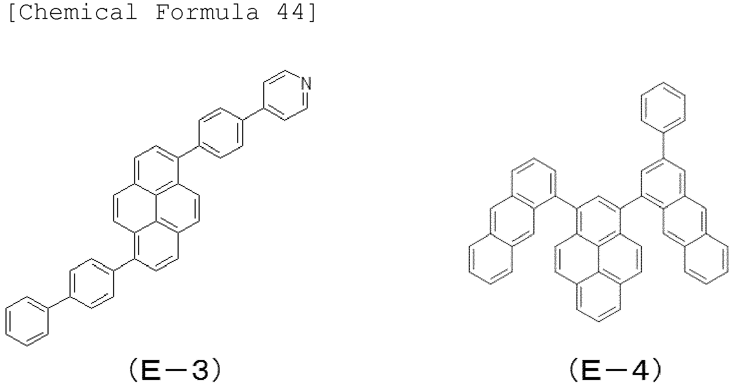

**[Table 2]**

| | Emissive material | | | Electron transporting layer | | Anode | External quantum efficiency (%) | Driving voltage (V) | Luminance half-life (h) |
|---|---|---|---|---|---|---|---|---|---|
| | Host material | Dopant material | Luminescent color | Compound | Donor compound | Metal | | | |
| Example 10 | | | Blue | Compound [1] | 2E-1 | Mg/Ag | 5.9 | 3.7 | 7,600 |
| Example 11 | | | Blue | Compound [2] | 2E-1 | Mg/Ag | 5.8 | 3.5 | 8,200 |
| Reference Example 12 | | | Blue | Compound [3] | 2E-1 | Mg/Ag | 5.7 | 4.3 | 7,700 |
| Reference Example 13 | | | Blue | Compound [4] | 2E-1 | Mg/Ag | 5.7 | 4.2 | 7,800 |
| Reference Example 14 | | | Blue | Compound [5] | 2E-1 | Mg/Ag | 5.8 | 4.0 | 7,800 |
| Reference Example 15 | | | Blue | Compound [6] | 2E-1 | Mg/Ag | 5.7 | 4.3 | 7,600 |
| Reference Example 16 | | | Blue | Compound [7] | 2E-1 | Mg/Ag | 5.9 | 4.3 | 7,500 |
| Reference Example 17 | | | Blue | Compound [8] | 2E-1 | Mg/Ag | 5.8 | 4.4 | 7,700 |
| Reference Example 18 | H-1 | D-1 | Blue | Compound [9] | 2E-1 | Mg/Ag | 5.8 | 4.2 | 7,500 |
| Reference Example 19 | | | Blue | Compound [10] | 2E-1 | Mg/Ag | 5.9 | 4.4 | 7,600 |
| Reference Example 20 | | | Blue | Compound [11] | 2E-1 | Mg/Ag | 5.8 | 4.3 | 7,500 |
| Reference Example 21 | | | Blue | Compound [12] | 2E-1 | Mg/Ag | 5.7 | 4.4 | 7,100 |
| Reference Example 22 | | | Blue | Compound [13] | 2E-1 | Mg/Ag | 5.6 | 4.1 | 7,300 |
| Reference Example 23 | | | Blue | Compound [14] | 2E-1 | Mg/Ag | 5.6 | 4.2 | 7,900 |
| Reference Example 24 | | | Blue | Compound [15] | 2E-1 | Mg/Ag | 5.7 | 4.3 | 8,000 |
| Reference Example 25 | | | Blue | Compound [16] | 2E-1 | Mg/Ag | 5.4 | 4.5 | 7,800 |
| Reference Example 26 | | | Blue | Compound [17] | 2E-1 | Mg/Ag | 5.8 | 4.3 | 7,700 |
| Reference Example 27 | | | Blue | Compound [18] | 2E-1 | Mg/Ag | 5.7 | 4.4 | 7,700 |
| Reference Example 28 | | | Blue | Compound [19] | 2E-1 | Mg/Ag | 5.8 | 4.4 | 7,700 |
| Reference Example 29 | | | Blue | Compound [20] | 2E-1 | Mg/Ag | 5.6 | 4.3 | 7,600 |
| Reference Example 30 | | | Blue | Compound [21] | 2E-1 | Mg/Ag | 5.5 | 4.5 | 7,000 |
| Reference Example 31 | | | Blue | Compound [22] | 2E-1 | Mg/Ag | 5.5 | 4.5 | 7,500 |
| Reference Example 32 | | | Blue | Compound [23] | 2E-1 | Mg/Ag | 5.6 | 4.6 | 7,100 |
| Reference Example 33 | | | Blue | Compound [24] | 2E-1 | Mg/Ag | 5.4 | 4.6 | 7,000 |
| Reference Example 34 | | | Blue | Compound [25] | 2E-1 | Mg/Ag | 5.4 | 4.4 | 7,500 |
| Example 35 | | | Blue | Compound [26] | 2E-1 | Mg/Ag | 5.6 | 3.9 | 7,800 |
| Example 36 | | | Blue | Compound [27] | 2E-1 | Mg/Ag | 5.9 | 3.6 | 7,900 |
| Example 37 | | | Blue | Compound [28] | 2E-1 | Mg/Ag | 5.7 | 3.8 | 8,100 |

**[Table 3]**

| | Emissive material | | | Electron transporting layer | | Anode | External quantum efficiency (%) | Driving voltage (V) | Luminance half-life (h) |
|---|---|---|---|---|---|---|---|---|---|
| | Host material | Dopant material | Luminescent color | Compound | Donor compound | Metal | | | |
| Example 38 | | | Blue | Compound [29] | 2E-1 | Mg/Ag | 5.4 | 3.6 | 7,700 |
| Example 39 | | | Blue | Compound [30] | 2E-1 | Mg/Ag | 5.2 | 3.3 | 6,100 |
| Example 40 | | | Blue | Compound [31] | 2E-1 | Mg/Ag | 5.8 | 3.6 | 8,200 |
| Example 41 | | | Blue | Compound | 2E-1 | Mg/Ag | 5.7 | 3.6 | 8,000 |
| Example 42 | | | Blue | Compound [33] | 2E-1 | Mg/Ag | 5.6 | 3.8 | 8,100 |
| Example 43 | | | Blue | Compound [34] | 2E-1 | Mg/Ag | 5.6 | 3.8 | 8,100 |
| Example 44 | | | Blue | Compound [35] | 2E-1 | Mg/Ag | 5.7 | 3.4 | 8,100 |
| Example 45 | H-1 | D-1 | Blue | Compound [36] | 2E-1 | Mg/Ag | 5.8 | 3.5 | 8,200 |
| Example 46 | | | Blue | Compound [37] | 2E-1 | Mg/Ag | 5.7 | 3.5 | 8,100 |
| Example 47 | | | Blue | Compound | 2E-1 | Mg/Ag | 5.4 | 3.6 | 7,200 |
| Example 48 | | | Blue | Compound [39] | 2E-1 | Mg/Ag | 5.8 | 3.8 | 8,000 |
| Example 49 | | | Blue | Compound [40] | 2E-1 | Mg/Ag | 5.7 | 3.7 | 8,000 |
| Example 50 | | | Blue | Compound [41] | 2E-1 | Mg/Ag | 5.9 | 3.7 | 7,900 |
| Example 51 | | | Blue | Compound [42] | 2E-1 | Mg/Ag | 5.9 | 3.8 | 8,000 |
| Example 52 | | | Blue | Compound [43] | 2E-1 | Mg/Ag | 5.8 | 3.6 | 7,800 |
| Example 53 | | | Blue | Compound [44] | 2E-1 | Mg/Ag | 5.8 | 3.7 | 8,000 |
| Example 54 | | | Blue | Compound [45] | 2E-1 | Mg/Ag | 5.8 | 3.8 | 7,600 |
| Example 55 | | | Blue | Compound [46] | 2E-1 | Mg/Ag | 5.6 | 4.0 | 8,200 |
| Example 56 | | | Blue | Compound [47] | 2E-1 | Mg/Ag | 5.5 | 4.0 | 7,900 |
| Example 57 | | | Blue | Compound [48] | 2E-1 | Mg/Ag | 5.9 | 4.2 | 7,800 |
| Example 58 | | | Blue | Compound [49] | 2E-1 | Mg/Ag | 6.0 | 3.9 | 7,700 |
| Example 59 | | | Blue | Compound | 2E-1 | Mg/Ag | 6.1 | 3.7 | 7,600 |
| Example 60 | | | Blue | Compound [51] | 2E-1 | Mg/Ag | 5.8 | 3.8 | 7,500 |
| Comparative Example 7 | | | Blue | E-1 | 2E-1 | Mg/Ag | 4.3 | 5.7 | 4,700 |
| Comparative Example 8 | | | Blue | E-2 | 2E-1 | Mg/Ag | 3.5 | 7.4 | 2,600 |
| Comparative Example 9 | | | Blue | E-3 | 2E-1 | Mg/Ag | 4.0 | 5.1 | 4,700 |
| Comparative Example 10 | | | Blue | E-4 | 2E-1 | Mg/Ag | 3.9 | 5.5 | 2,000 |

### Examples 61 to 71

In the same manner as in Example 1, expect that each of materials shown in Table 4 was used as a host material, a dopant material and a material of an electron transporting layer, light emitting devices were produced. The results are shown in Table 4. In Table 4, (H-2) to (H-8) and (D-2) to (D-10) are the following compounds.

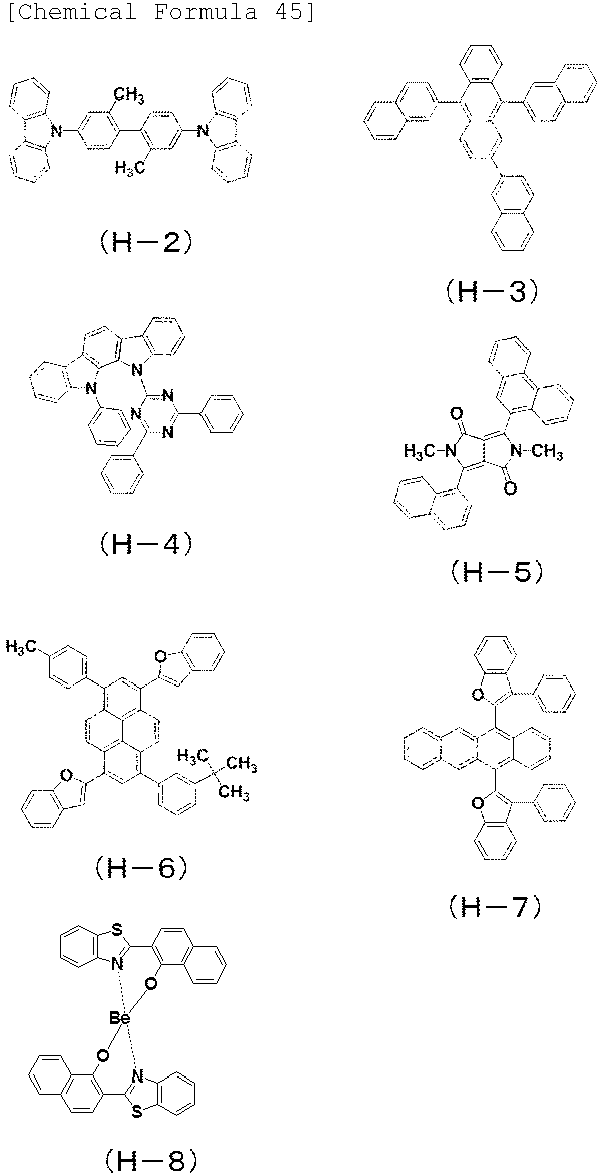

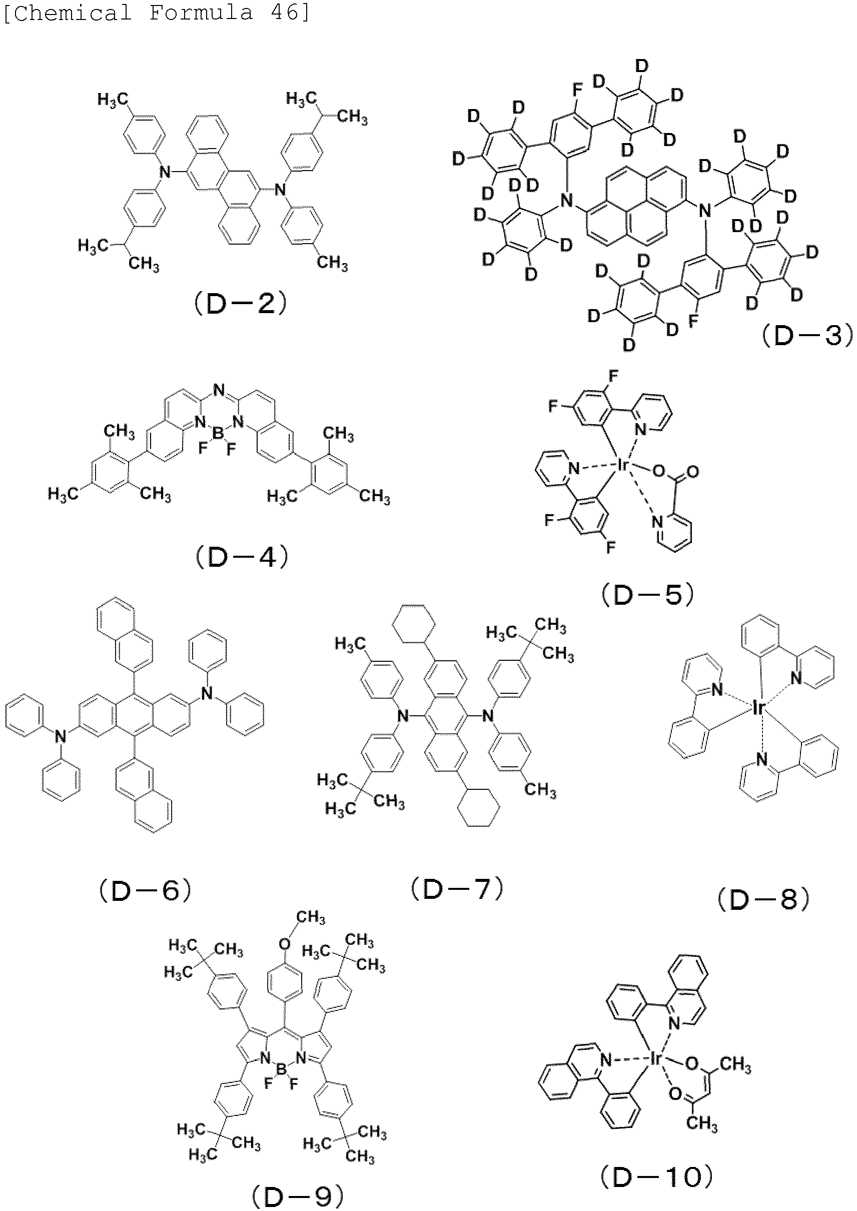

**[Table 4]**

| | Emissive material | | | Electron transporting layer | | Anode | External quantum efficiency (%) | Driving voltage (V) | Luminanc e half-life (h) |
|---|---|---|---|---|---|---|---|---|---|
| | Host material | Dopant material | Luminescent color | Compound | Donor compound | Metal | | | |
| Example 61 | H-1 | D-2 | Blue | Compound [1] | 2E-1 | Al | 5.9 | 4.3 | 6,900 |
| Example 62 | | D-3 | Blue | | | Al | 6.0 | 4.2 | 7,000 |
| Example 63 | | D-4 | Blue | | | Al | 5.6 | 4.1 | 6,800 |
| Example 64 | H-2 | D-5 | Blue | | | Al | 8.2 | 4.9 | 4,000 |
| Example 65 | H-3 | D-6 | Green | Compound [1] | 2E-1 | Al | 7.5 | 4.1 | 6,400 |
| Example 66 | | D-7 | Green | | | Al | 7.2 | 4.2 | 5,500 |
| Example 67 | H-4 | D-8 | Green | | | Al | 12.0 | 5.3 | 6,600 |
| Example 68 | H-5 | D-9 | Red | Compound [1] | 2E-1 | Al | 5.6 | 4.4 | 5,100 |
| Example 69 | H-6 | | Red | | | Al | 5.7 | 4.3 | 7,300 |
| Example 70 | H-7 | | Red | | | Al | 6.0 | 4.3 | 7,400 |
| Example 71 | H-8 | D-10 | Red | | | Al | 11.8 | 5.2 | 7,200 |

### Example 72

In the same manner as in Example 1, except that the compound [1] was used as a host material and tris(8-quinolinolato)aluminum(III) (Alq₃) was used as a material of an electron transporting layer, a light emitting device was produced. This light emitting device was direct-current driven at 10 mA/cm². As a result, the obtained light emitting device enables low driving voltage and high-efficiency light emission, and is also excellent in durability, and exhibited a driving voltage of 4.8 V, an external quantum efficiency of 4.9%, and luminance half-life of 7,900 hours.

### Examples 73 and 74, Reference Examples 75 to 80

In the same manner as in Example 72, expect that each of materials shown in Table 5 was used as a host material, light emitting devices were produced. The results are shown in Table 5.

### Comparative Examples 11 to 14

In the same manner as in Example 72, expect that each of materials shown in Table 5 was used as a host material, light emitting devices were produced. The results are shown in Table 5. In Table 5, (H-9) is the following compound.

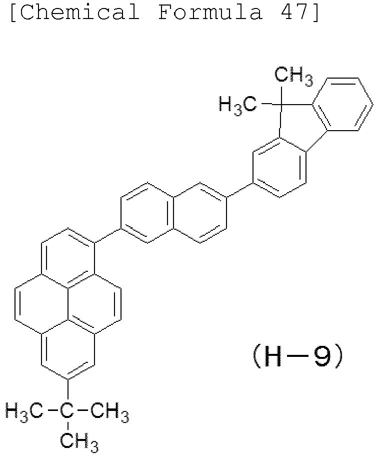

**[Table 5]**

| | Emissive material | | | Electron transporting layer | Anode | External quantum efficiency (%) | Driving voltage (V) | Luminance half-life (h) |
|---|---|---|---|---|---|---|---|---|
| | Host material | Dopant material | Luminescent color | Compound | Metal | | | |
| Example 73 | Compound [1] | D-1 | Blue | Alq3 | Al | 4.9 | 4.8 | 7,900 |
| Example 74 | Compound [2] | | Blue | Alq3 | Al | 4.8 | 4.7 | 7,800 |
| Reference Example 75 | Compound [3] | | Blue | Alq3 | Al | 5.1 | 5.0 | 8,000 |
| Reference Example 76 | Compound [12] | | Blue | Alq3 | Al | 4.4 | 5.3 | 7,100 |
| Reference Example 77 | Compound [14] | | Blue | Alq3 | Al | 5.3 | 5.0 | 8,300 |
| Reference Example 78 | Compound [18] | | Blue | Alq3 | Al | 5.5 | 5.1 | 7,900 |
| Reference Example 79 | Compound [22] | | Blue | Alq3 | Al | 5.8 | 5.5 | 8,400 |
| Reference Example 80 | Compound [25] | | Blue | Alq3 | Al | 5.8 | 5.4 | 8,300 |
| Comparative Example 11 | E-1 | | Blue | Alq3 | Al | 3.1 | 5.8 | 4,200 |
| Comparative Example 12 | E-2 | | Blue | Alq3 | Al | 3.2 | 5.9 | 3,800 |
| Comparative Example 13 | E-4 | | Blue | Alq3 | Al | 3.0 | 6.3 | 3,000 |
| Comparative Example 14 | H-9 | | Blue | Alq3 | Al | 3.5 | 6.3 | 3,500 |

### [Industrial Applicability]

The present invention provides a light emitting device material capable of providing an organic thin-film light emitting device which enables high-efficiency light emission and low-voltage driving, and is also excellent in durability, and a light emitting device using the same. The light emitting device material of the present invention can be preferably used for an electron transporting layer or an emissive layer of a light emitting device.

## Claims

1. A light emitting device material containing a compound represented by the following general formula (1): 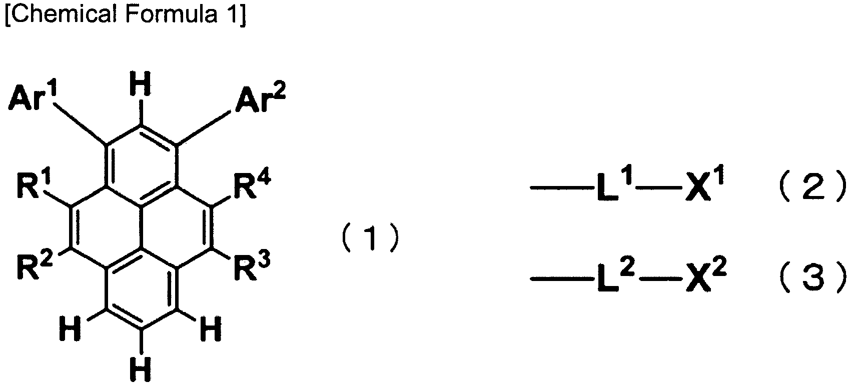 wherein R¹ to R⁴ may be the same or different, and are each selected from the group consisting of hydrogen, an alkyl group, a cycloalkyl group, a heterocyclic group, an alkenyl group, a cycloalkenyl group, an alkynyl group, an alkoxy group, an alkylthio group, an arylether group, an arylthioether group, an aryl group, a heteroaryl group, halogen, a carbonyl group, a carboxyl group, an oxycarbonyl group, a carbamoyl group, an amino group, a silyl group, and -P(=O)R⁵R⁶; R⁵ and R⁶ are each an aryl group or a heteroaryl group, and adjacent substituents may form a ring; Ar¹ is a group represented by the general formula (2); Ar² is a group represented by the general formula (3); L¹ and L² are each a single bond, an arylene group, or a heteroarylene group, and each may be the same or different, provided that when L¹ and L² are each a naphthalenylene group, L¹ and L² each is not linked to pyrene at the 1-position of a naphthalene ring; and X¹ and X² are each an aryl group or a heteroaryl group, and each may be the same or different, provided that when L¹ is a single bond, X¹ is not a 1-naphthyl group and, when L² is a single bond, X² is not a 1-naphthyl group, wherein at least one of X¹ and X² is an aromatic heterocyclic group containing electron-accepting nitrogen.

2. The light emitting device material according to claim 1, wherein when X¹ is a 2-pyridyl group, X² is selected from groups other than the 2-pyridyl group.

3. The light emitting device material according to claim 1 or 2, wherein L¹ and L² are each a single bond, a phenylene group, a biphenylene group, or a naphthalenylene group.

4. The light emitting device material according to any one of claims 1 to 3, wherein Ar¹ and Ar² are different groups.

5. A light emitting device comprising an anode, a cathode, and an organic layer existing between the anode and the cathode, the light emitting device emitting light from electric energy, wherein the organic layer contains the light emitting device material according to any one of claims 1 to 4.

6. The light emitting device according to claim 5, wherein the organic layer includes an electron transporting layer, and the electron transporting layer contains the light emitting device material according to any one of claims 1 to 4.

7. The light emitting device according to claim 6, wherein the electron transporting layer further contains a donor compound.

8. The light emitting device according to claim 7, wherein the donor compound is an alkali metal, an inorganic salt containing an alkali metal, a complex of an alkali metal and an organic substance, an alkali earth metal, an inorganic salt containing an alkali earth metal, or a complex of an alkali earth metal and an organic substance.

9. The light emitting device according to claim 8, wherein the donor compound is a complex of an alkali metal and an organic substance, or a complex of an alkali earth metal and an organic substance.

10. The light emitting device according to claim 5, wherein the organic layer includes an emissive layer, and the emissive layer contains the light emitting device material according to any one of claims 1 to 4.

## Patentansprüche

1. Lichtemittierendes Vorrichtungsmaterial enthaltend eine Verbindung, welche durch die folgende allgemeine Formel (1) dargestellt ist: 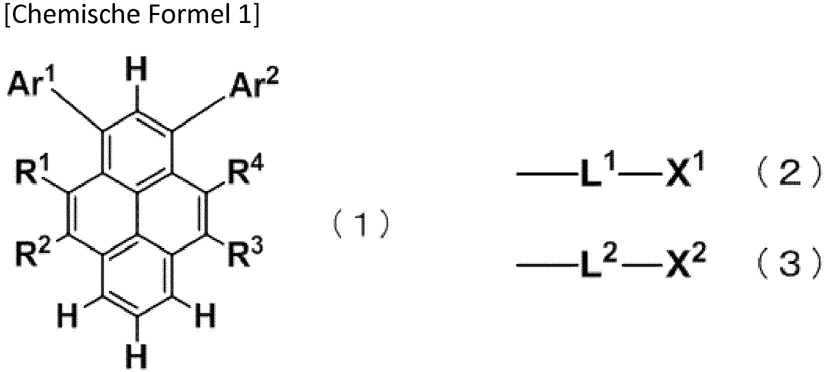 wobei R¹ bis R⁴ gleich oder verschieden sein können, und jeweils ausgewählt sind aus der Gruppe bestehend aus Wasserstoff, einer Alkylgruppe, einer Cycloalkylgruppe, einer heterocyclischen Gruppe, einer Alkenylgruppe, einer Cycloalkenylgruppe, einer Alkinylgruppe, einer Alkoxygruppe, einer Alkylthiogruppe, einer Arylethergruppe, einer Arylthioethergruppe, einer Arylgruppe, einer Heteroarylgruppe, einem Halogen, einer Carbonylgruppe, einer Carboxylgruppe, einer Oxycarbonylgruppe, einer Carbamoylgruppe, einer Aminogruppe, einer Silylgruppe, und -P(=O)R⁵R⁶; wobei R⁵ und R⁶ jeweils eine Arylgruppe oder eine Heteroarylgruppe sind, und benachbarte Substituenten einen Ring bilden können; Ar¹ eine Gruppe, welche durch die allgemeine Formel (2) dargestellt ist, ist; Ar² eine Gruppe, welche durch die allgemeine Formel (3) dargestellt ist, ist; L¹ und L² jeweils eine Einfachbindung, eine Arylengruppe oder eine Heteroarylengruppe sind, und jeweils gleich oder unterschiedlich sein können, sofern, wenn L¹ und L² jeweils eine Naphthalinylengruppe sind, L¹ und L² jeweils nicht an Pyren an der 1-Position des Naphthalinringes gebunden sind; und X¹ und X² jeweils eine Arylgruppe oder eine Heteroarylgruppe sind, und jeweils gleich oder unterschiedlich sein können, sofern, wenn L¹ eine Einfachbindung ist, X¹ keine 1-Naphthylgruppe ist, und wenn L² eine Einfachbindung ist, X² keine 1-Naphthylgruppe ist, wobei mindestens einer von X¹ und X² eine aromatische heterocyclische Gruppe enthaltend einen elektronenakzeptierenden Stickstoff ist.

2. Lichtemittierendes Vorrichtungsmaterial nach Anspruch 1, wobei, wenn X¹ eine 2-Pyridylgruppe ist, X² ausgewählt ist aus Gruppen, welche sich von der 2-Pyridylgruppe unterscheiden.

3. Lichtemittierendes Vorrichtungsmaterial nach Anspruch 1 oder 2, wobei L¹ und L² jeweils eine Einfachbindung, eine Phenylengruppe, eine Biphenylengruppe oder eine Naphthalinylengruppe sind.

4. Lichtemittierendes Vorrichtungsmaterial nach einem der Ansprüche 1 bis 3, wobei Ar¹ und Ar² unterschiedliche Gruppen sind.

5. Lichtemittierende Vorrichtung umfassend eine Anode, eine Kathode, und eine organische Schicht, welche zwischen der Anode und der Kathode existiert, wobei die lichtemittierende Vorrichtung Licht durch elektrische Energie emittiert, wobei die organische Schicht das lichtemittierende Vorrichtungsmaterial nach einem der Ansprüche 1 bis 4 enthält.

6. Lichtemittierende Vorrichtung nach Anspruch 5, wobei die organische Schicht eine Elektronentransportschicht enthält, und die Elektronentransportschicht das lichtemittierende Vorrichtungsmaterial nach einem der Ansprüche 1 bis 4 enthält.

7. Lichtemittierendes Vorrichtungsmaterial nach Anspruch 6, wobei die Elektronentransportschicht ferner eine Donorverbindung enthält.

8. Lichtemittierende Vorrichtung nach Anspruch 7, wobei die Donorverbindung ein Alkalimetall, ein anorganisches Salz enthaltend ein Alkalimetall, ein anorganisches Salz enthaltend ein Alkalimetall, ein Komplex eines Alkalimetalls und einer organischen Substanz, ein Erdalkalimetall, ein anorganisches Salz enthaltend ein Erdalkalimetall, oder ein Komplex eines Erdalkalimetalls und einer organischen Substanz ist.

9. Lichtemittierende Vorrichtung nach Anspruch 8, wobei die Donorverbindung ein Komplex eines Alkalimetalls und einer organischen Substanz oder ein Komplex eines Erdalkalimetalls und einer organischen Substanz ist.

10. Lichtemittierende Vorrichtung nach Anspruch 5, wobei die organische Schicht eine emissive Schicht enthält, und die emissive Schicht das lichtemittierende Vorrichtungsmaterial nach einem der Ansprüche 1 bis 4 enthält.

## Revendications

1. Matériau de dispositif électroluminescent contenant un composé représenté par la formule générale (1) suivante : 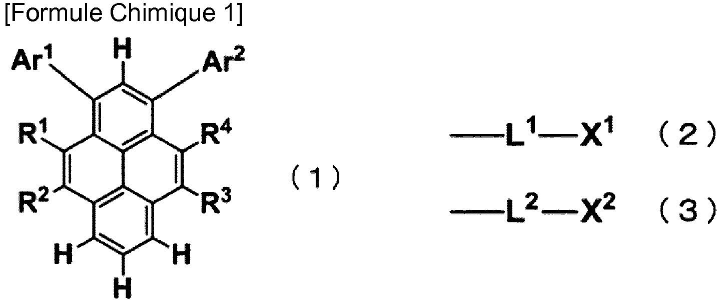 où R¹ à R⁴ peuvent être identiques ou différents, et sont choisis chacun dans le groupe constitué d'un atome d'hydrogène, d'un groupe alkyle, d'un groupe cycloalkyle, d'un groupe hétérocyclique, d'un groupe alcényle, d'un groupe cycloalcényle, d'un groupe alcynyle, d'un groupe alcoxy, d'un groupe alkylthio, d'un groupe aryléther, d'un groupe arylthioéther, d'un groupe aryle, d'un groupe hétéroaryle, d'un atome d'halogène, d'un groupe carbonyle, d'un groupe carboxyle, d'un groupe oxycarbonyle, d'un groupe carbamoyle, d'un groupe amino, d'un groupe silyle, et de -P(=O)R⁵R⁶ ; R⁵ et R⁶ représentent chacun un groupe aryle ou un groupe hétéroaryle, et des substituants adjacents peuvent former un noyau ; Ar¹ représente un groupe représenté par la formule générale (2) ; Ar² représente un groupe représenté par la formule générale (3) ; L¹ et L² représentent chacun une liaison simple, un groupe arylène ou un groupe hétéroarylène, et peuvent chacun être identiques ou différents, à condition que lorsque L¹ et L² représentent chacun un groupe naphtalénylène, chacun de L¹ et L² n'est pas lié au pyrène à la position 1 d'un noyau naphtalène ; et X¹ et X² représentent chacun un groupe aryle ou un groupe hétéroaryle, et peuvent chacun être identiques ou différents, à condition que lorsque L¹ représente une liaison simple, X¹ ne représente pas un groupe 1-naphtyle et, lorsque L² représente une liaison simple, X² ne représente pas un groupe 1-naphtyle, où au moins l'un de X¹ et X² représente un groupe hétérocyclique aromatique contenant un atome d'azote accepteur d'électrons.

2. Matériau de dispositif électroluminescent selon la revendication 1, dans lequel lorsque X¹ représente un groupe 2-pyridyle, X² est choisi parmi des groupes autres que le groupe 2-pyridyle.

3. Matériau de dispositif électroluminescent selon la revendication 1 ou 2, dans lequel L¹ et L² représentent chacun une liaison simple, un groupe phénylène, un groupe biphénylène ou un groupe naphtalénylène.

4. Matériau de dispositif électroluminescent selon l'une quelconque des revendications 1 à 3, dans lequel Ar¹ et Ar² représentent des groupes différents.

5. Dispositif électroluminescent comprenant une anode, une cathode et une couche organique existant entre l'anode et la cathode, le dispositif électroluminescent émettant de la lumière provenant d'une énergie électrique, où la couche organique contient le matériau de dispositif électroluminescent selon l'une quelconque des revendications 1 à 4.

6. Dispositif électroluminescent selon la revendication 5, dans lequel la couche organique comporte une couche de transport d'électrons, et la couche de transport d'électrons contient le matériau de dispositif électroluminescent selon l'une quelconque des revendications 1 à 4.

7. Dispositif électroluminescent selon la revendication 6, dans lequel la couche de transport d'électrons contient en outre un composé donneur.

8. Dispositif électroluminescent selon la revendication 7, dans lequel le composé donneur est un métal alcalin, un sel inorganique contenant un métal alcalin, un complexe d'un métal alcalin et d'une substance organique, un métal alcalino-terreux, un sel inorganique contenant un métal alcalino-terreux, ou un complexe d'un métal alcalino-terreux et d'une substance organique.

9. Dispositif électroluminescent selon la revendication 8, dans lequel le composé donneur est un complexe d'un métal alcalin et d'une substance organique, ou un complexe d'un métal alcalino-terreux et d'une substance organique.

10. Dispositif électroluminescent selon la revendication 5, dans lequel la couche organique comporte une couche émissive, et la couche émissive contient le matériau de dispositif électroluminescent selon l'une quelconque des revendications 1 à 4.
